(19) Europäisches Patentamt
European Patent Office
Office européen des brevets

(11) EP 4 382 091 A1

(12) EUROPEAN PATENT APPLICATION

(43) Date of publication:
**12.06.2024 Bulletin 2024/24**

(21) Application number: **22212442.2**

(22) Date of filing: **09.12.2022**

(51) International Patent Classification (IPC):
***A61K 9/00*** (2006.01) ***A61K 9/107*** (2006.01)
***A61K 41/00*** (2020.01) ***A61K 51/04*** (2006.01)
***A61K 51/12*** (2006.01) ***C07C 323/52*** (2006.01)
***A61K 47/10*** (2017.01) ***A61K 47/20*** (2006.01)
***C08G 65/333*** (2006.01) ***C08G 65/334*** (2006.01)
***C08G 65/337*** (2006.01)

(52) Cooperative Patent Classification (CPC):
**A61K 9/0026; A61K 9/1075; A61K 41/0038; A61K 47/10; A61K 47/20; A61K 51/0478; A61K 51/1227; C07C 323/52; C08G 65/33396; C08G 65/3348; C08G 65/337**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Commissariat à l'Energie Atomique et aux Energies Alternatives**
  **75015 Paris (FR)**
- **Université Paris-Saclay**
  **91190 Saint-Aubin (FR)**
- **Centre National de la Recherche Scientifique**
  **75016 Paris (FR)**
- **Institut National de la Santé et de la Recherche Médicale (INSERM)**
  **75013 Paris (FR)**

(72) Inventors:
- **DORIS, Eric**
  **91191 Gif-sur-Yvette (FR)**
- **GRAVEL, Edmond**
  **91191 Gif-sur-Yvette (FR)**
- **GODEL, Sophia**
  **91191 Gif-sur-Yvette (FR)**
- **TRUILLET, Charles**
  **91401 Orsay (FR)**
- **PORCEL, Erika**
  **91405 Orsay (FR)**
- **JAMGOTCHIAN, Lucie**
  **69003 Lyon (FR)**

(74) Representative: **Cabinet Nony**
**11 rue Saint-Georges**
**75009 Paris (FR)**

# (54) PERFLUORINATED MICELLES AND MEDICAL USES THEREOF

(57) The invention relates to the fields of radiotherapy adjuvants, anti-cancer treatment, and tissue hypoxia prevention or treatment. Herein the inventors report the development of perfluorinated nanoscale micelles for the *in vivo* uptake and transport of oxygen to tissues, in particular tumor tissues, in order to overcome hypoxia to treat and/or prevent cancer in a subject, and potentiate radiotherapy.

EP 4 382 091 A1

## Description

### TECHNICAL FIELD

The invention relates to the fields of radiotherapy adjuvants, anti-cancer treatment, and tissue hypoxia prevention or treatment.

### BACKGROUND OF THE INVENTION

Almost half of all cancer patients will undergo radiotherapy in the course of their medical treatment. Unfortunately, the success of tumor treatment with ionizing radiation is hampered by a number of radioresistance phenomena in cancer cells.

In particular, hypoxia, *i.e.* the decrease in oxygen levels in the tumor tissue, can significantly reduce the efficacy of radiotherapy, as detailed in Baumann et al. (Nat. Rev. Cancer, 2016, 16, 234). In fact, at the tumor site, the local decrease in oxygen levels limits the amount of reactive oxygen species (ROS) that can be produced by the radiation, thus reducing the impact of radiation therapy. Moreover, DNA strand breaks consequent to ionizing radiation exposure react with molecular dioxygen, leading to permanent damage in the cell, as detailed in Suwa et al. (Exp. Mol. Med., 2021, 53, 1029).

It has thus been proposed that the delivery of oxygen to the tumor site would potentiate the local effect of radiation by increasing the production of reactive oxygen species and the amount of DNA damages, without inducing additional toxicity for the surrounding healthy tissues.

There are several ways to overcome tumor hypoxia, through a strategy called "radiosensitization", as detailed in Wang et al. (Cancers, 2019, 11, 112). In a non-exhaustive manner, those solutions may include:

i) the normalization of the tumor vasculature to restore sufficient blood supply;
ii) the increase of oxygen in the blood stream by use of external sources (*e.g.* micro-bubbles);
iii) the use of nanoparticles incorporating $O_2$-generating peroxides or exhibiting catalase-like activity;
iv) the use of vectors to transport oxygen from the blood to the tumor tissue.

Jamgotchian et al. (Nanoscale, 2021, 13, 2373) reports superfluorinated micellar probes for diagnostic and drug delivery purposes. Yet, this document is silent on their ability to transport and release $O_2$ *in vivo,* let alone in specific tumor tissues.

WO2018/187236 teaches emulsion-based formulations for use in drug delivery, comprising semi-fluorinated block copolymers. This document is also silent on their ability to transport and release $O_2$ *in vivo,* let alone in specific tumor tissues.

Yet, re-oxygenation of tissues, particularly tumor tissues, is still a major challenge in the global context of medical care, and especially cancer therapy.

As part of the latter strategy, the use of perfluorinated compounds has shown promising results. Perfluorocarbons (PFC) have the ability to efficiently solubilize gases, including oxygen, as reported in Krafft, M. P. (Curr. Opin. Pharmacol., 2020, 53, 117) and in Jägers et al. (Eur. J. Physiol., 2021, 473:139-150). This property is due to the low level of association of PFC molecules with each other (unlike organic solvents or water), resulting in cavities that can accommodate gases.

However, while PFCs may improve the transport of oxygen they do not necessarily provide selective delivery to the tumor site. Moreover, while PFCs may possess the ability to solubilize $O_2$, they are not necessarily able to retain the oxygen *in vivo,* in a predictable, targeted, durable and efficient manner.

WO2010/077671 teaches perfluorocarbon emulsions for use as artificial oxygen carrier, prepared from a mixture of PFC and PEO-b-PCL copolymers, characterized by a droplet size in the range of 250 to 450 nm. However, this document does not provide *in vivo* evidence of oxygen transport and release in tissues, let alone in tumor tissue specifically.

Wang et al. (Biomater. Sci., 2019, 6, 3096-3107) teaches fluorinated and non-fluorinated polymeric assemblies prepared respectively from amphiphilic polymers PFOC-PEI and OC-PEI, which are characterized by a droplet size in the range of 106 to 141 nm. This document reports their use as photosensitizers, and evidences $O_2$ release *in vivo,* in a tumor-bearing mouse model, following photodynamic therapy.

There is thus a general need for novel oxygen carriers, in particular in areas such as cancer therapeutics, blood substitutes, organ preservation and diving disease.

There is thus a general need for compositions and methods for increasing the level of oxygen, either in the blood stream or in specific tissues, in a targeted manner.

There is thus a general need for solutions and methods for treating or preventing tissue hypoxia; either in a

general manner, or in the specific content of cancer treatment.

There is thus still a general need for novel compositions which can be of use in radiotherapy or photodynamic cancer therapy, and in particular in the context of radiosensitization.

The invention has for purpose to meet the above-mentioned needs.

## SUMMARY OF THE INVENTION

According to a **first main embodiment,** the invention relates to a biocompatible micelle composition, for use as a medicament, characterized in that:

(i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons;
(ii) the micelle has an average hydrodynamic diameter equal or inferior to 40 nm.

In particular, the invention relates to a biocompatible micelle composition, as defined above, for use in radiotherapy.

In particular, the invention relates to the biocompatible micelle composition, as defined above, for use in a method for treating or preventing tissue hypoxia in a patient.

According to **a second main embodiment,** the invention relates to an amphiphilic molecule, comprising at least one hydrophobic perfluorinated moiety linked to a hydrophilic metal complexing moiety; characterized in that the hydrophilic metal complexing moiety comprises desferrioxamine or a derivative thereof.

According to **a third main embodiment,** the invention relates to a method for preparing an amphiphilic molecule, as defined above, said amphiphilic molecule comprising at least one hydrophobic perfluorinated moiety linked to a hydrophilic metal complexing moiety; characterized in that the hydrophilic metal complexing moiety comprises desferrioxamine or a derivative thereof.

According to **a fourth main embodiment,** the invention relates to a biocompatible micelle composition, the micelle comprising:

(i) a plurality of amphiphilic molecules comprising at least one hydrophobic perfluorinated moiety linked to at least one hydrophilic moiety;
(ii) a plurality of amphiphilic molecules comprising at least one hydrophobic perfluorinated moiety linked to at least one hydrophilic metal complexing moiety comprising desferrioxamine or a derivative thereof.

According to **a fifth main embodiment,** the invention relates to a kit comprising

(i) a plurality of amphiphilic molecules comprising at least one hydrophobic perfluorinated moiety linked to at least one hydrophilic moiety;
(ii) a plurality of amphiphilic molecules comprising at least one hydrophobic perfluorinated moiety linked to at least one hydrophilic metal complexing moiety comprising desferrioxamine or a derivative thereof.

According to **a sixth main embodiment,** the invention relates to a method for preparing a micelle composition, comprising a step of:

a) bringing into contact (i) a plurality of amphiphilic molecules comprising at least one hydrophobic perfluorinated moiety linked to at least one hydrophilic moiety, and (ii) a plurality of amphiphilic molecules comprising at least one hydrophobic perfluorinated moiety linked to at least one hydrophilic metal complexing moiety comprising desferrioxamine or a derivative thereof;
b) optionally ultra-sonicating the composition obtained at step a);

thereby preparing the micelle composition.

## BRIEF DESCRIPTION OF THE FIGURES

**Figure 1: Synthesis of PFTD-PEG:** i) **3**, **4**, NaH, THF, at room temperature for 24 h, 66%.

**Figure 2: Synthesis of PFTD-DFO:** i) TsCl, NaOH, THF, 0 °C to room temp.; ii) HSTrt, NaOH, EtOH/toluene 1:1, room temp.; iii) TsCl, DCM, room temp.; iv) **4**, NaH, THF, 75 °C; v) TFA, TIPS, $CH_2Cl_2$, room temp; vi) succinimidyl 2-iodoacetate, DIPEA, DMF/$H_2O$ 10:1, room temp.; vii) $K_2CO_3$, DMSO, room temperature.

**Figure 3A: Release of oxygen from different micelle colloids injected in degassed water.** In X-axis, time (s). In Y-axis, $O_2$ release expressed in $\mu$mol $L^{-1}$. Each curve corresponds to a different micelle concentration, respectively from bottom to top: water; 9 mM SDS; 3.8 mM Triton X100); 7.6 mM Triton X100); 1.9 mM PFTD-PEG; 3.8 mM PFTD-PEG; 7.6 mM PFTD-PEG.

**Figure 3B: Evolution of the oxygen content in various $O_2$-saturated aqueous dispersions upon ambient air-equilibration.** In X-axis, time ($10^3$ s). In Y-axis, Relative $O_2$ content. Each curve corresponds to a different micelle concentration, respectively from top to bottom: 3.8 mM PFTD-PEG, 3.8 mM Triton X100, 9 mM SDS and water.

**Figure 4: Assessment of the internalization pathway of PFTD-PEG micelles in B16F10 cells by incubation under different inhibitory conditions**. In X-axis from left to right: control, Amiloride, Genistein, Sucrose, and storage at 4°C. In Y-axis: Mean Fluorescence Intensity (MFI). The control refers to cells brought into contact with micelles PFTD-PEG loaded with 1% w/w DiD without any inhibitor + **** $p < 0.0001$

**Figure 5: Toxicity assessment of PFTD-PEG micelles on B16F10 cells at varying concentrations. A)** In X-axis from left to right: control, 0.01 mg/mL, 0.1 mg/mL, 0.5 mg/mL, 1 mg/mL, Staurosporine 1$\mu$M. In Y-axis Cell survival measured by MTS assay (%); **B**) Clonogenic assay. In X-axis from left to right: control, 0.1 mg/mL, 0.25 mg/mL, 0.5 mg/mL, 1 mg/mL, Staurosporine 1$\mu$M. In Y-axis surviving fraction.

**Figure 6: Effect of different irradiation doses on the survival of B16F10 cells** In X-axis: irradiation dose (Gy). In Y-axis: survival fraction. The two curves include BF16F10 cells incubated with PFTD-PEG micelles at 0.25 mg/ml (triangle) or with simple culture medium (control).

**Figure 7. Biodistribution of radiolabeled micelles after intravenous injection to mice bearing sub-cutaneous B16F10 tumors.** In X-axis for each group of bars, from the left to the right of a group of bars, respectively : (i) after 1h of injection, (ii) 4 h, (iii) 24 h, (iv) 48 h, (v) 72 h, (vi) 168 h. In Y-axis: % ID $cm^{-3}$. Each group of bars corresponds, from left to right, to the biodistribution in blood, liver, spleen, tumor, kidney, bone, lung, muscle, brain.

**Figure 8. UV and DLS profiles of micellar PFTD-PEG suspension containing 1% DiD w/w. 8A. UV profile** with absorbance in Y-axis and wavelength (nm) in X-axis. 8B. DLS profile with volume (ml) in Y-axis and diameter (nmà in X-axis.

## DETAILED DESCRIPTION

Herein the inventors report the development of perfluorinated nanoscale micelles for the *in vivo* uptake and transport of oxygen to tissues, in particular tumor tissues, in order to overcome hypoxia, to treat and/or prevent cancer in a subject, and potentiate radiotherapy.

Without wishing to be bound by the theory, the inventors are of the opinion that the micelles according to the present disclosure possess the ability to target the tumor and to locally deliver their oxygen cargo, thus enhancing the production of toxic reactive oxygen species under ionizing radiation treatment.

The micelle can be characterized by a perfluorinated core for $O_2$ solubilization, and a hydrophilic surface (e.g. a polyethylene glycol surface) for *in vivo* biocompatibility and passive tissue (e.g. tumor tissue) targeting.

Without wishing to be bound by the theory, the inventors are of the opinion that the micelle formulations according to the disclosure also share hydrodynamic properties which are compatible with efficient $O_2$ transport, cell internalization and tissue oxygenation.

They are of the opinion that low hydrodynamic size prevents them from being immobilized *in vivo* by the extracellular collagen network, and thus permits their diffusion deeply in the tumor tissue.

Thus, the micellar formulation according to the present disclosure allows for internalization within tumor cells by endocytosis, to deliver oxygen as close as possible to cell material, in particular the genetic material, and thus potentiate the effect of reactive species generated by ionizing radiation.

Hence, the inventors provide experimental evidence that **(i)** the perfluorinated micelles were not cytotoxic in the absence of ionizing radiations, that **(ii)** they possess the capacity to translocate from the extracellular medium to the interior of the cells, where the transported oxygen should be able to exert its radiosensitizing effect, and that **(iii)** the micelles accumulate within B16F10 murine melanoma cells, in particular through endocytosis, and more particularly

caveolae- and clathrin-dependant pathways.

Advantageously, the effect of gamma rays was significantly increased in the presence of micelles, with a sensitizer enhancement ratio of 1.23. It is worth mentioning that, despite their organic nature and the absence of high Z elements in their composition, the developed perfluorinated micelles produce a radiosensitization of the exposed tumor cells (radiation enhancement ratio of 1.30 at 2 Gy) which is equivalent or even superior to that generated by metallic nanoparticles.

According to particular embodiments, the micelles or micelle compositions according to the present invention are not loaded with a therapeutic compound.

According to particular embodiments, the micelles or micelle compositions according to the present invention comprise a fluorescent or phosphorescent molecule.

According to particular embodiments, the micelles or micelle compositions according to the present invention do not comprise a radioactive isotope and/or a high-Z element.

In order to complete the study and evaluate their *in vivo* behavior, perfluorinated micelles were also successfully labelled with $^{89}Zr$ thanks to the introduction of metal-complexing moieties, in particular desferrioxamine (DFO) complexing groups, in their structure. The radiolabeled micelles were then injected to tumor bearing mice and their distribution was monitored by PET imaging over several days. During this biodistribution study, a tumor accumulation of perfluorinated micelles, resulting from EPR effect, was demonstrated with a maximum at 24 h (7.1 $\pm$ 0.7 %DI $cm^{-3}$). The PET-derived distribution profile of perfluorinated micelles within the mouse organism also highlighted liver uptake and hepatobiliary excretion of the objects.

The *in vitro* radiosensitizing effect on cancer cells coupled to the high tumor accumulation demonstrated *in vivo* make PFTD-PEG micelles highly promising nanovectors for the potentiation of radiotherapy. The fact that these objects can be easily radiolabeled adds to their attractiveness, as they can be viewed as theranostic agents.

Main Embodiments

According to a **first main embodiment,** the invention relates to a biocompatible micelle composition, for use as a medicament, characterized in that:

(i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons, in particular having a molecular weight of at least 1000 Daltons, more particularly having a molecular weight of at least 1500 Daltons, preferably having a molecular weight of at least 2000 Daltons;

(ii) the micelle has an average hydrodynamic diameter equal or inferior to 40 nm, in particular equal or inferior to 20 nm, more particularly equal or inferior to 16 nm, preferably equal or inferior to 13 nm.

In particular, the invention relates to a biocompatible micelle composition, for use in radiotherapy, characterized in that:

(i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons, in particular having a molecular weight of at least 1000 Daltons, more particularly having a molecular weight of at least 1500 Daltons, preferably having a molecular weight of at least 2000 Daltons;

(ii) the micelle has an average hydrodynamic diameter equal or inferior to 40 nm, in particular equal or inferior to 20 nm, more particularly equal or inferior to 16 nm, preferably equal or inferior to 13 nm.

According to some other particular embodiments, the invention relates to a biocompatible micelle composition, for use in photodynamic therapy, characterized in that:

(i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons, in particular having a molecular weight of at least 1000 Daltons, more particularly having a molecular weight of at least 1500 Daltons, preferably having a molecular weight of at least 2000 Daltons;

(ii) the micelle has an average hydrodynamic diameter equal or inferior to 40 nm, in particular equal or inferior to 20 nm, more particularly equal or inferior to 16 nm, preferably equal or inferior to 13 nm.

In particular, the invention relates to a biocompatible micelle composition, for use in a method for treating or preventing tissue hypoxia in a patient, characterized in that:

(i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons, in particular having a molecular weight of at least 1000 Daltons, more particularly having a molecular weight of at least 1500 Daltons, preferably having a molecular weight of at least 2000 Daltons;
(ii) the micelle has an average hydrodynamic diameter equal or inferior to 40 nm, in particular equal or inferior to 20 nm, more particularly equal or inferior to 16 nm, preferably equal or inferior to 13 nm.

According to some particular embodiments, the patient may be a patient suffering from cancer or any other proliferative disorder.
According to some particular embodiments, the cancer or proliferative disorder is not a blood or blood-related cancer.
Hence, according to some other particular embodiments, the invention relates to a biocompatible micelle composition, for use in treating and/or preventing tumor tissue hypoxia in a patient, characterized in that:

(i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons, in particular having a molecular weight of at least 1000 Daltons, more particularly having a molecular weight of at least 1500 Daltons, preferably having a molecular weight of at least 2000 Daltons;
(ii) the micelle has an average hydrodynamic diameter equal or inferior to 40 nm, in particular equal or inferior to 20 nm, more particularly equal or inferior to 16 nm, preferably equal or inferior to 13 nm.

According to some other particular embodiments, the invention relates to a biocompatible micelle composition, for use in treating and/or preventing cancer in a patient, characterized in that:

(i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons, in particular having a molecular weight of at least 1000 Daltons, more particularly having a molecular weight of at least 1500 Daltons, preferably having a molecular weight of at least 2000 Daltons;
(ii) the micelle has an average hydrodynamic diameter equal or inferior to 40 nm, in particular equal or inferior to 20 nm, more particularly equal or inferior to 16 nm, preferably equal or inferior to 13 nm.

According to some embodiments, the micelles may be characterized in that the amphiphilic molecules have a molecular weight of at least 500 Daltons, and the micelle has an average hydrodynamic diameter equal or inferior to 20 nm.
According to some embodiments, the micelles may be characterized in that the amphiphilic molecules have a molecular weight of at least 500 Daltons, and the micelle has an average hydrodynamic diameter equal or inferior to 16 nm.
According to some embodiments, the micelles may be characterized in that the amphiphilic molecules have a molecular weight of at least 500 Daltons, and the micelle has an average hydrodynamic diameter equal or inferior to 13 nm.
According to some embodiments, the micelles may be characterized in that the amphiphilic molecules have a molecular weight of at least 1000 Daltons, and the micelle has an average hydrodynamic diameter equal or inferior to 20 nm.
According to some embodiments, the micelles may be characterized in that the amphiphilic molecules have a molecular weight of at least 1000 Daltons, and the micelle has an average hydrodynamic diameter equal or inferior to 16 nm.
According to some embodiments, the micelles may be characterized in that the amphiphilic molecules have a molecular weight of at least 1000 Daltons, and the micelle has an average hydrodynamic diameter equal or inferior to 13 nm.
According to some embodiments, the micelles may be characterized in that the amphiphilic molecules have a molecular weight of at least 1500 Daltons, and the micelle has an average hydrodynamic diameter equal or inferior to 20 nm.
According to some embodiments, the micelles may be characterized in that the amphiphilic molecules have a molecular weight of at least 1500 Daltons, and the micelle has an average hydrodynamic diameter equal or inferior to 16 nm.

According to some embodiments, the micelles may be characterized in that the amphiphilic molecules have a molecular weight of at least 1500 Daltons, and the micelle has an average hydrodynamic diameter equal or inferior to 13 nm.

According to some embodiments, the micelles may be characterized in that the amphiphilic molecules have a molecular weight of at least 2000 Daltons, and the micelle has an average hydrodynamic diameter equal or inferior to 20 nm.

According to some embodiments, the micelles may be characterized in that the amphiphilic molecules have a molecular weight of at least 2000 Daltons, and the micelle has an average hydrodynamic diameter equal or inferior to 16 nm.

According to some embodiments, the micelles may be characterized in that the amphiphilic molecules have a molecular weight of at least 2000 Daltons, and the micelle has an average hydrodynamic diameter equal or inferior to 13 nm.

According to particular embodiments, the micelles may be characterized in that the hydrophilic moiety has a molecular weight of at least 500 Daltons; for example in that the hydrophilic moiety has a molecular weight of at least 1000 Daltons; for example in that the hydrophilic moiety has a molecular weight of at least 1500 Daltons; for example in that the hydrophilic moiety has a molecular weight of at least 2000 Daltons.

According to some embodiments, the micelles may be characterized in that the hydrophilic moiety has a molecular weight of at least 500 Daltons, and the micelle has an average hydrodynamic diameter equal or inferior to 20 nm.

According to some embodiments, the micelles may be characterized in that the hydrophilic moiety has a molecular weight of at least 500 Daltons, and the micelle has an average hydrodynamic diameter equal or inferior to 16 nm.

According to some embodiments, the micelles may be characterized in that the hydrophilic moiety has a molecular weight of at least 500 Daltons, and the micelle has an average hydrodynamic diameter equal or inferior to 13 nm.

According to some embodiments, the micelles may be characterized in that the hydrophilic moiety has a molecular weight of at least 1000 Daltons, and the micelle has an average hydrodynamic diameter equal or inferior to 20 nm.

According to some embodiments, the micelles may be characterized in that the hydrophilic moiety has a molecular weight of at least 1000 Daltons, and the micelle has an average hydrodynamic diameter equal or inferior to 16 nm.

According to some embodiments, the micelles may be characterized in that the hydrophilic moiety has a molecular weight of at least 1000 Daltons, and the micelle has an average hydrodynamic diameter equal or inferior to 13 nm.

According to some embodiments, the micelles may be characterized in that the hydrophilic moiety has a molecular weight of at least 1500 Daltons, and the micelle has an average hydrodynamic diameter equal or inferior to 20 nm.

According to some embodiments, the micelles may be characterized in that the hydrophilic moiety has a molecular weight of at least 1500 Daltons, and the micelle has an average hydrodynamic diameter equal or inferior to 16 nm.

According to some embodiments, the micelles may be characterized in that the hydrophilic moiety has a molecular weight of at least 1500 Daltons, and the micelle has an average hydrodynamic diameter equal or inferior to 13 nm.

According to some embodiments, the micelles may be characterized in that the hydrophilic moiety has a molecular weight of at least 2000 Daltons, and the micelle has an average hydrodynamic diameter equal or inferior to 20 nm.

According to some embodiments, the micelles may be characterized in that the hydrophilic moiety has a molecular weight of at least 2000 Daltons, and the micelle has an average hydrodynamic diameter equal or inferior to 16 nm.

According to some embodiments, the micelles may be characterized in that the hydrophilic moiety has a molecular weight of at least 2000 Daltons, and the micelle has an average hydrodynamic diameter equal or inferior to 13 nm.

According to particular embodiments the micelles may be characterized in that the amphiphilic molecule is a linear or ramified molecule.

According to particular embodiments the micelles may be characterized in that the perfluorinated hydrophobic moiety is a linear or ramified perfluorinated hydrophobic moiety.

According to particular embodiments the micelles may be characterized in that the hydrophilic moiety comprises or consists of a polymer, such as a homopolymer or a heteropolymer, in particular a polyoxygenated polymer, and/or a polymer selected from: synthetic polymers, natural polymers and zwitterionic polymers; and more particularly a polymer selected from the group consisting of: polyethylene glycol (PEG), poly(oligo(ethylene glycol) methyl ether methacrylate (POEGMA), polyglycerols (PGs), poly(oxazolines) (POX), poly(hydroxypropylmethacrylate) (PHPMA), poly(2-hydroxyethylmethacrylate) (PHEMA), poly(N-(2-hydroxypropyl)methacrylamide) (HPMA), poly(vinylpyrrolidone) (PVP), poly(N,N-dimethyl acrylamide) (PDMA), and poly(N-acryloylmorpholine) (PAcM), poly(acrylamide), poly(methacrylamide), poly(N-acryloylmorpholine), glycosaminoglycan (GAGs), polyhyaluronic acid (pHA), polyheparin, polysialic acid (pSA), polyaminoacid, polypeptide, poly(carboxybetaine acrylamide) (pCBAA), poly(carboxybetaine methacrylate), poly(sulfobetaine methacrylate), poly(methacryloyloxyethylphosphorylcholine), poly(vinyl-pyridiniopropanesulfonate), poly(carboxybetaine) (pCB), poly(sulfobetaine) (pSB), phosphobetaine-based, poly(2-methacryloyloxyethylphosphorylcholine) (PMPC), poly(β-amino ester) (PAE) based polymers.

According to a particular embodiment, the biocompatible micelle composition may be characterized in that the hydrophilic moiety comprises or consists of a polyoxygenated polymer, more particularly a polyethylene glycol (PEG)

polymer, for example a polyethylene glycol (PEG) polymer having a molecular weight of at least 350 Daltons, for example a polyethylene glycol (PEG) polymer having a molecular weight of at least 500 Daltons, for example a polyethylene glycol (PEG) polymer having a molecular weight of at least 1000 Daltons, for example a polyethylene glycol (PEG) polymer having a molecular weight of at least 1500 Daltons, for example a polyethylene glycol (PEG) polymer having a molecular weight of at least 2000 Daltons.

According to a particular embodiment, the biocompatible micelle composition may be characterized in that the hydrophilic moiety comprises a polyethylene glycol (PEG) containing at least 8 ethoxy monomers; for example at least 9, 10, 11, 12, 13, 14, 15, 16, 17, 18, 19, 20, 21, 22, 23, 24, 25, 26, 27, 28, 29, 30, 40, 41, 42, 43, 44, 45, 46, 47, 48, 49, 50 ethoxy monomers.

According to a particular embodiment, the biocompatible micelle composition may be characterized in that the hydrophilic moiety comprises a polyethylene glycol (PEG) containing 100 ethoxy monomers or less than 100 ethoxy monomers; for example less than 99, 98, 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50 ethoxy monomers.

According to a particular embodiment, the biocompatible micelle composition may be characterized in that the hydrophilic moiety comprises a polyethylene glycol (PEG) containing between 8 and 100 ethoxy monomers; in particular containing between 30 and 60 ethoxy monomers, and more particularly containing between 40 and 50 ethoxy monomers.

According to a particular embodiment, the hydrophobic perfluorinated moiety comprises or consists of at least one selected from: a perfluorinated substituted $C_1$-$C_{30}$ alkyl moiety, perfluorinated unsubstituted $C_1$-$C_{30}$ alkyl moiety.

According to exemplified embodiments, the hydrophobic perfluorinated moiety comprises or consists of at least one selected from: a linear perfluorinated substituted $C_1$-$C_{30}$ alkyl moiety, a linear perfluorinated unsubstituted $C_1$-$C_{30}$ alkyl moiety.

According to a particular embodiment, the biocompatible micelle composition may be characterized in that the plurality of amphiphilic molecules comprises or consists of:

[A] - $[Linker]_m$ - [B];

or

$[A]_{term}$ - [A] - $[Linker1]_m$- [B] - $[B]_{term}$;

[A]term - [Linkerl]m - [A] - [Linker2]n - [B] - [Linker3]o - [B]term;

wherein m and n and o are, independently, 0 or 1;
wherein [A] is a perfluorinated hydrophobic moiety, in particular a $C_1$-$C_{30}$ substituted or unsubstituted perfluoroalkyl moiety;
wherein [A]term is a hydrophobic terminal moiety, in particular a perfluorinated hydrophobic moiety, for example selected from: $-CF_3$ or $-RCF_3$ with R being a $C_1$-$C_{30}$ alkyl;
wherein [B] is a hydrophilic moiety, in particular a hydrophilic polymer, for example a polyethylene glycol (PEG) polymer ;
wherein [B]term is a hydrophilic terminal moiety;
wherein [Linker1] and [Linker2] and [Linker3] are the same linker regions or different linker regions; for example linker regions comprising or consisting of a substituted or unsubstitued alkyl (e.g. a $C_1$-$C_{30}$ alkyl) or a substituted or unsubstituted alkoxy (e.g. a $C_1$-$C_{30}$ alkoxy).

According to particular embodiments, [B]term is selected from an hydroxy-terminated or amino-terminated or siloxane-terminated functional group.

According to particular embodiments, $[B]_{term}$ may be selected from a functional group comprising or consisting of: -OH or $-OCH_3$ or $-COCH_3$ or -COOH or $-CH_2COOH$ or $-NH_3$.

According to a particular embodiment, the biocompatible micelle composition may be characterized in that the plurality of amphiphilic molecules comprises or consists of:

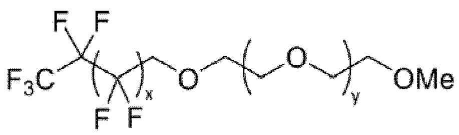
;

wherein **x** is equal or superior to 1;
wherein **y** is equal or superior to 1;
with "Me" referring to a methyl ($-CH_3$).

According to a particular embodiment, the biocompatible micelle composition may be characterized in that the plurality of amphiphilic molecules comprises or consists of:

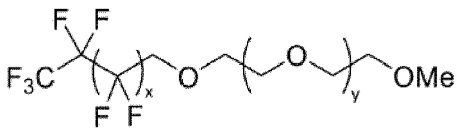
;

wherein **x** is equal or superior to 8;
wherein **y** is equal or superior to 8.

According to a particular embodiment, the biocompatible micelle composition may be characterized in that the plurality of amphiphilic molecules comprises or consists of:

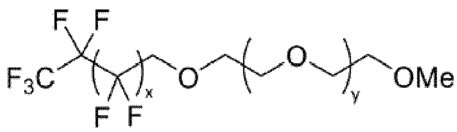
;

wherein **x** ranges from 1 to 100;
wherein **y** ranges from 1 to 100.

According to a particular embodiment, the biocompatible micelle composition may be characterized in that the plurality of amphiphilic molecules comprises or consists of:

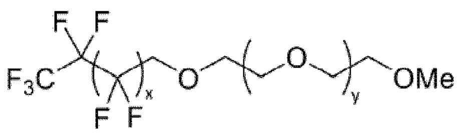
;

wherein **x** ranges from 8 to 15;
wherein **y** ranges from 8 to 100.

According to a particular embodiment, the biocompatible micelle composition may be characterized in that the plurality of amphiphilic molecules comprises or consists of:

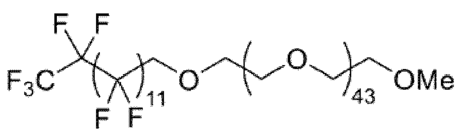
.

According to some of those embodiments, the biocompatible micelle composition may be characterized in that the plurality of amphiphilic molecules comprises or consists of:

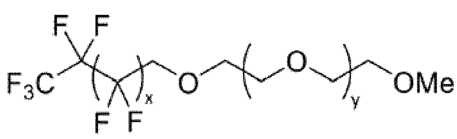
;

wherein the ratio x/y is equal or inferior to 1;
with x and y being separately equal or superior to 1, for example equal or superior to 8.

According to a particular embodiment, the biocompatible micelle composition may be characterized in that the amphiphilic molecule is a block copolymer, a di-block copolymer or a tri-block copolymer, comprising at least one hydrophobic perfluorinated block moiety linked to at least one hydrophilic block moiety.

According to a particular embodiment, the biocompatible micelle composition may be characterized in that the plurality of amphiphilic molecules comprises a hydrophobic perfluorinated moiety linked to a hydrophilic metal complexing moiety; in particular a hydrophilic metal complexing moiety comprising desferrioxamine or a derivative thereof.

According to particular embodiments, the amphiphilic molecule may be characterized in that it comprises a hydrophobic perfluorinated moiety linked to a hydrophilic metal complexing moiety comprising desferrioxamine or a derivative thereof. Hence, according to particular embodiments, the biocompatible micelle composition may be characterized in that the plurality of amphiphilic molecules comprises:

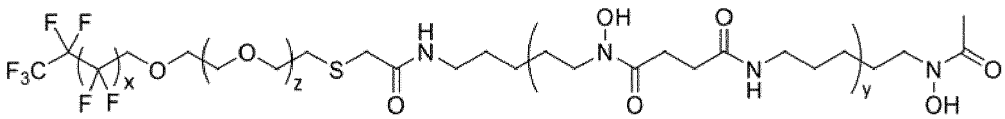

;

wherein **x** is equal or superior to 1;
wherein **y** is equal or superior to 2;
wherein **z** is equal or superior to 1.

According to particular embodiments, the biocompatible micelle composition may be characterized in that the plurality of amphiphilic molecules comprises:

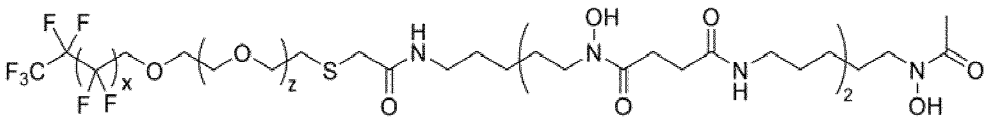

;

wherein **x** is equal or superior to 1;
wherein **z** is equal or superior to 1.

According to particular embodiments, the biocompatible micelle composition may be characterized in that the plurality of amphiphilic molecules comprises or consists of:

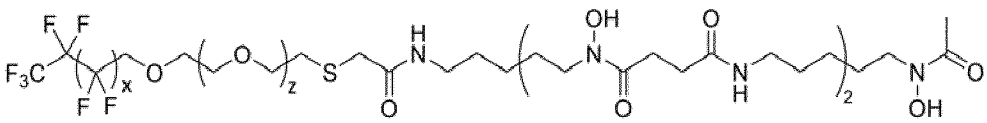

;

wherein **x** ranges from 8 to 15;
wherein **z** ranges from 0 to 10.

According to particular embodiments, the biocompatible micelle composition may be characterized in that the plurality of amphiphilic molecules comprises or consists of:

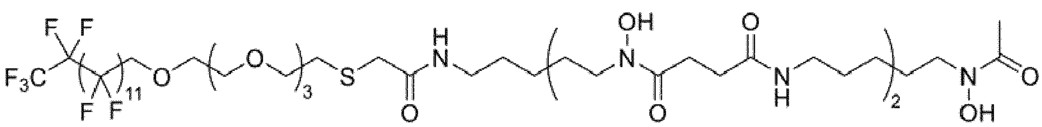

.

According to **a second main embodiment,** the invention relates to an amphiphilic molecule, comprising at

least one hydrophobic perfluorinated moiety linked to a hydrophilic metal complexing moiety; characterized in that the hydrophilic metal complexing moiety comprises desferrioxamine or a derivative thereof.

According to particular embodiments, the biocompatible micelle composition may be characterized in that the plurality of amphiphilic molecules comprises:

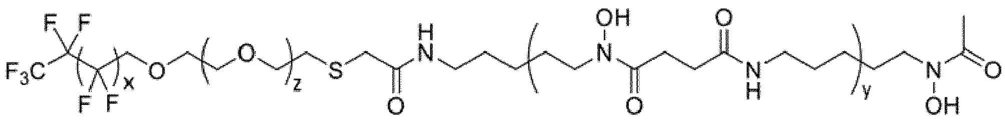;

wherein **x** is equal or superior to 1;
wherein **y** is equal or superior to 2;
wherein **z** is equal or superior to 1.

According to a preferred embodiment, the invention relates to the amphiphilic molecule as defined above, which consists of:

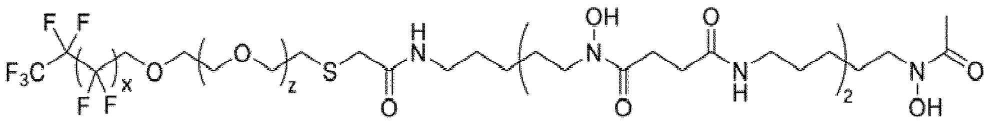;

wherein **x** ranges from 8 to 15;
wherein **z** ranges from 0 to 10.

According to a preferred embodiment, the invention relates to the amphiphilic molecule as defined above, which consists of:

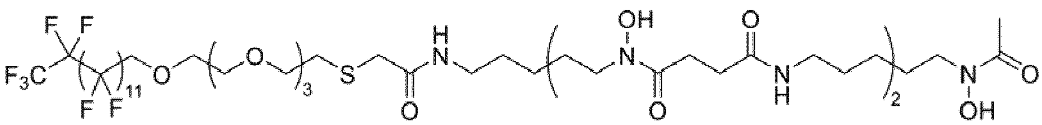.

According to **a third main embodiment,** the invention relates to a method for preparing an amphiphilic molecule, as defined above, said amphiphilic molecule comprising at least one hydrophobic perfluorinated moiety linked to a hydrophilic metal complexing moiety; characterized in that the hydrophilic metal complexing moiety comprises desferrioxamine or a derivative thereof.

It will be readily understood hereafter that compositions as such, and kits as such, referring to amphiphilic molecules may thus comprise either one of the plurality of amphiphilic mocules which were previously described.

According to **a fourth main embodiment,** the invention relates to a biocompatible micelle composition, the micelle comprising:

(i) a plurality of amphiphilic molecules comprising at least one hydrophobic perfluorinated moiety linked to at least one hydrophilic moiety;
(ii) a plurality of amphiphilic molecules comprising at least one hydrophobic perfluorinated moiety linked to at least one hydrophilic metal complexing moiety comprising desferrioxamine or a derivative thereof.

According to particular embodiments, the invention relates to a biocompatible micelle composition, the micelle comprising:

(i) a plurality of amphiphilic molecules comprising at least one hydrophobic perfluorinated moiety linked to at least one hydrophilic moiety, and of general formula:

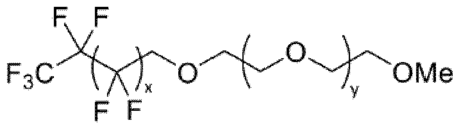

wherein **x** is equal or superior to 1;
wherein **y** is equal or superior to 1;
with "Me" referring to a methyl ($-CH_3$);

(ii) a plurality of amphiphilic molecules comprising at least one hydrophobic perfluorinated moiety linked to at least one hydrophilic metal complexing moiety comprising desferrioxamine or a derivative thereof.

According to particular embodiments, the invention relates to a biocompatible micelle composition, the micelle comprising:

(i) a plurality of amphiphilic molecules comprising at least one hydrophobic perfluorinated moiety linked to at least one hydrophilic moiety;
(ii) a plurality of amphiphilic molecules comprising at least one hydrophobic perfluorinated moiety linked to at least one hydrophilic metal complexing moiety comprising desferrioxamine or a derivative thereof, and of general formula:

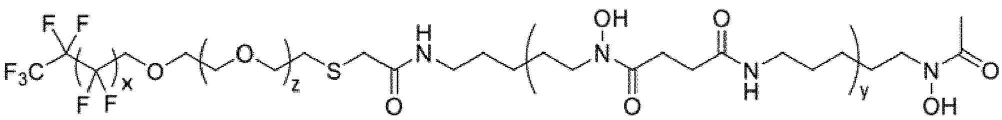

wherein **x** is equal or superior to 1;
wherein **y** is equal or superior to 2;
wherein **z** is equal or superior to 1.

According to **a fifth main embodiment,** the invention relates to a kit comprising

(i) a plurality of amphiphilic molecules comprising at least one hydrophobic perfluorinated moiety linked to at least one hydrophilic moiety;
(ii) a plurality of amphiphilic molecules comprising at least one hydrophobic perfluorinated moiety linked to at least one hydrophilic metal complexing moiety comprising desferrioxamine or a derivative thereof.

According to particular embodiments, the invention relates to a kit comprising:

(ii) a plurality of amphiphilic molecules comprising at least one hydrophobic perfluorinated moiety linked to at least one hydrophilic moiety, and of general formula:

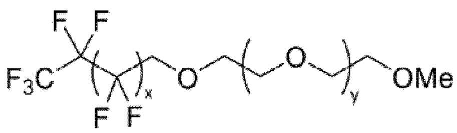

wherein **x** is equal or superior to 1;
wherein **y** is equal or superior to 1;
with "Me" referring to a methyl ($-CH_3$);

(ii) a plurality of amphiphilic molecules comprising at least one hydrophobic perfluorinated moiety linked to at least one hydrophilic metal complexing moiety comprising desferrioxamine or a derivative thereof.

According to particular embodiments, the invention relates to a kit comprising:

(iii) a plurality of amphiphilic molecules comprising at least one hydrophobic perfluorinated moiety linked to at least one hydrophilic moiety;

(iv) a plurality of amphiphilic molecules comprising at least one hydrophobic perfluorinated moiety linked to at least one hydrophilic metal complexing moiety comprising desferrioxamine or a derivative thereof, and of general formula:

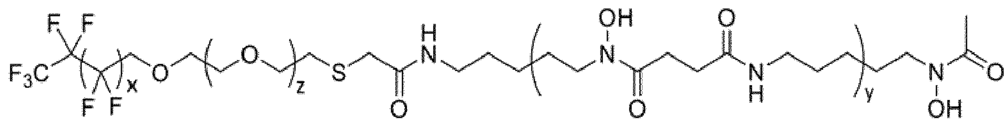;

wherein **x** is equal or superior to 1;
wherein **y** is equal or superior to 2;
wherein **z** is equal or superior to 1.

According to particular embodiments of the said compositions or kits, the amounts of the corresponding plurality of amphiphilic molecules may vary; either in an isolated form in a kit or when combined altogether in order to form a micelle and/or a biocompatible micelle composition.

According to particular embodiments of the said compositions and kits, amphiphilic molecules (i) and amphiphilic molecules (ii) may be present in a 5:1 to 15:1 **(i)/(ii)** mass ratio; in particular in a 8:1 to 10:1 **(i)/(ii)** mass ratio.

According to **a sixth main embodiment,** the invention relates to a method for preparing a micelle composition, comprising a step of:

a) bringing into contact (i) a plurality of amphiphilic molecules comprising at least one hydrophobic perfluorinated moiety linked to at least one hydrophilic moiety, and (ii) a plurality of amphiphilic molecules comprising at least one hydrophobic perfluorinated moiety linked to at least one hydrophilic metal complexing moiety comprising desferrioxamine or a derivative thereof;

b) optionally ultra-sonicating the composition obtained at step a);

thereby preparing the micelle composition.

Preferably, step a) is achieved in conditions suitable for the preparation of a micelle comprising the said plurality of amphiphilic molecules.

According to most preferred embodiments, step a) is achieved in conditions wherein the concentration of each amphiphilic molecule is at or above the relevant critical micelle concentration.

According to exemplified embodiments, step a) is achieved with molecule (i) and molecule (ii) in a 5:1 to 15:1 (i)/(ii) mass ratio; in particular in a 8:1 to 10:1 (i)/(ii) mass ratio.

The present disclosure further relates to a therapeutic treatment or prevention method comprising administering, to a subject in need thereof, and in particular to a subject suffering from cancer or at risk thereof, and/or a subject suffering from tissue hypoxia (e.g. tumor tissue hypoxia) a biocompatible micelle composition characterized in that:

(i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons;

(ii) the micelle has an average hydrodynamic diameter equal or inferior to 40 nm.

According to a particular embodiment, the method further comprises a step of into contact the subject with an ionizing radiation and/or a light source, in particular bringing into contact a tumor tissue of the subject with an ionizing radiation.

According to a particular embodiment, the present disclosure relates to a radiotherapy or photodynamic therapy in a subject in need thereof, comprising a step of administering a biocompatible micelle composition characterized in that:

(i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons;

(ii) the micelle has an average hydrodynamic diameter equal or inferior to 40 nm.

According to a particular embodiment, the present disclosure relates to a radiotherapy method in a subject in need thereof, comprising a step of:

**a**) administering a biocompatible micelle composition characterized in that:

(i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons;
(ii) the micelle has an average hydrodynamic diameter equal or inferior to 40 nm.

**b**) bringing into contact the subject with a ionizing radiation, in particular bringing into contact a tumor tissue of the subject with a ionizing radiation.

According to a particular embodiment, the present disclosure relates to a photodynamic therapy in a subject in need thereof, comprising a step of:

**a**) administering a biocompatible micelle composition characterized in that:

(iii) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons;
(iv) the micelle has an average hydrodynamic diameter equal or inferior to 40 nm.

**b**) bringing into contact the subject with a light source, in particular bringing into contact a tumor tissue of the subject with a light source.

According to a particular embodiment, the present disclosure relates to a radiotherapy or photodynamic therapy method in a subject in need thereof, comprising a step of bringing into contact the subject with a ionizing radiation or a light source ; characterized in that the subject in need thereof has been administered a biocompatible micelle composition according to the disclosure:

(i) the micelle comprising a plurality of amphiphilic molecules, said amphiphilic molecules comprising at least one hydrophobic perfluorinated moiety linked to at least one hydrophilic moiety, said amphiphilic molecules having a molecular weight of at least 500 Daltons;
(ii) the micelle having an average hydrodynamic diameter equal or inferior to 40 nm.

## General definitions

As used in this specification and the appended claims, the singular forms "**a**", "**an**" and "**the**" include plural referents unless the content clearly dictates otherwise.

As used in this specification and the appended claims, the term "**at least one**", may thus include one, or "**more than one**". Accordingly, the terms "**a plurality of"** or "**more than one**" may thus include « **two** » or « **two or more** ».

The term "**comprise**" is to be interpreted as specifying the presence of the stated features, integers, steps or components, but not precluding the presence of one or more other features, integers, steps or components, or group thereof. Also, it may specify strictly the stated features, integers, steps or components, and therefore in such case it may be replaced with "**consist of**".

As used herein, the term "**emulsion**" refers to a mixture of two or more immiscible substances, such as a mixture of two immiscible liquids. Emulsions are a type of colloid that comprise at least one dispersed phase dispersed in a continuous phase. Emulsions are broadly defined as two immiscible phases in which a first phase is dispersed within a second phase, such as a two- phase system in which one liquid is dispersed throughout a second liquid in the form of small droplets. The two phases of an emulsion are generally referred to as the continuous phase and the dispersed phase, with the dispersed phase typically present as a smaller volume percentage. A dispersion of oil in water is referred to as an oil-in-water (o/w) emulsion. For o/w emulsions the emulsifying agent is typically more soluble in the aqueous phase. The reverse emulsion, water-in-oil, is abbreviated w/o and is stabilized by surfactants that are more stable in the oil phase. In an aqueous emulsion, the continuous phase is an aqueous solution. Emulsions include nanoemulsions comprising nanoscale droplets of one immiscible liquid dispersed within another.

As used herein, the term « **nanoemulsion** » is a heterogeneous system composed of one immiscible liquid dispersed as nanonascale droplets within another liquid, where the average droplet diameter is below 1000 nm.

As used herein, the term « **micelle** » refers to the aggregate of amphiphilic molecules or « **surfactants** », dispersed in a liquid. Micelles can thus be defined as colloidal dispersions from amphiphilic molecules (e.g. polymers or block copolymers) with a hydrophobic tail and a hydrophilic head. Such micelles form in the liquid after reaching the

corresponding critical micelle concentration (or CMC) of the amphiphile/surfactant(s). Hence, the term may encompass both micelles assembled from one type of amphiphile and micelles assembled from a plurality of distinct amphiphiles ; such as those micelles assembled from two or more distinct amphiphiles. Micelles may be characterized from other types of aggregates by their organisation into one or more ordered layers, and in particular mono-layers. Unless specified otherwise, the term may encompass micelles having all ranges of hydrodynamic size, in particular those having an hydrodynamic size at or below 40 nm, such as at or below 39, 38, 37, 36, 35, 34, 33, 32, 31, 30, 29, 28, 27, 26, 25, 24, 23, 22, 21, 20, 19, 18, 17, 16, 15, 14 or 13nm.

As used herein, the term « **amphiphile** » refers to molecules and/or components (e.g., functional groups/moieties,polymers, and blocks of block polymers, etc.) of molecules having at least one hydrophilic moiety and at least one hydrophobic moiety, said hydrophilic and hydrophobic moieties being optionally linked by one or more linker regions. In particular, the term may refer to such molecules and/or components of such molecules, which are able to associate or co-associate in order to form micelles at or above a given CMC.

In particular, the term "**amphiphilic molecule**" according to the present disclosure may refer to a molecule, or component thereof, comprising at least one hydrophobic perfluorinated moiety linked to at least one hydrophilic moiety, of the following general formula:

$$[\text{hydrophobic perfluorinated moiety}] - [\text{linker1}]_x - [\text{hydrophobic moiety}]_y - [\text{linker2}]_z - [\text{hydrophilic moiety}];$$

with x and y and z being independently 0 or 1, said [linker1] and [linker 2] being the same or different.

Alternatively, the term "**amphiphilic molecule**" according to the present disclosure may refer to a molecule, or component thereof, comprising at least one hydrophobic perfluorinated moiety linked to at least one hydrophilic moiety, of the following general formula:

$$[\text{hydrophobic moiety}] - [\text{linker1}]_x - [\text{hydrophobic perfluorinated moiety}]_y - [\text{linker2}]_z - [\text{hydrophilic moiety}];$$

with x and y and z being independently 0 or 1, said [linker1] and [linker 2] being the same or different.

As used herein, the term "**linker**" refers to any group or moiety, in particular any functional group, which is compatible with the inclusion of the amphiphilic molecule according to the disclosure into the corresponding biocompatible micelle. Examples of such linker regions may consist of those selected from: carbonyl groups, alkyl groups, -O-, -O(CH2)$_e$-, $-(CH_2)_eO-$, $-(CH_2)_e-$, $-(CH_2)_eO(CH_2)-$, $-(CH_2)_eNR^1(CH_2)_f$,$-(CH_2)_eOCONR_2(CH_2)_f$-, $-(CH_2)_eCONR^3(CH_2)_f$-, $-(CH_2)_eNR^4COO(CH_2)_f$-, - $(CH_2)_eNR^5CO(CH_2)$- or $-(CH_2)_eNR^6CONR^7(CH_2)$- ; where each of $R_1$-$R_7$ is independently hydrogen, methyl, or $C_1$-$C_5$ alkyl; and where each of e and f is independently an integer selected from the range of 0 to 5. In some embodiments, the linking moiety is - $(CH_2)_eO(CH_2)_f$ -, where e and f are each independently 1.

Examples of functional groups which may act as linkers include those selected from: alkyl, alkenyl, alkynyl, alkylamides, carboxylic esters, carbonates, carbamates, carbamides, amides, sulfamides, sulfides, disulfides, sulfonic esters, phosphoric esters, ketone.

As used herein, the term "**hydrophilic**"refers to molecules and/or components (e.g., functional groups/moieties, polymers, and blocks of block polymers, etc.) of molecules having at least one hydrophilic group, and the term **"hydrophobic**" refers to molecules and/or components (e.g., functional groups/moieties, polymers, and blocks of block copolymers etc.) of molecules having at least one hydrophobic group.

Hydrophilic molecules or components thereof tend to have ionic and/or polar groups, and hydrophobic molecules or components thereof tend to have nonionic and/or nonpolar groups. Hydrophilic molecules or components thereof tend to participate in stabilizing interactions with an aqueous solution, including hydrogen bonding and dipoledipole interactions. Examples of hydrophilic molecules may comprise PEG polymers.

Hydrophobic molecules or components tend not to participate in stabilizing interactions with an aqueous solution and, thus often cluster together in an aqueous solution to achieve a more stable thermodynamic state.

As used herein, the terms "**average droplet size**" or "**average hydrodynamic size**" or "**average hydrodynamic diameter"** are used interchangeably. The average droplet size of a particle or droplet or micelle can be determined by any dynamic light scattering (DLS) technique, for example a dynamic light scattering measurement by a Cordouan Vasco-Flex apparaturs, equipped with a 450 nm laser, at ambient temperature (e.g. at 20°C or 25°C).

As used herein, the term **"semi-fluorinated"** refers to chemical compounds having at least one fluorine atom, for example molecules having at least one carbon - fluorine bond.

As used herein, the term "**fluorocarbons**" refer to chemical compounds that contain at least one carbon-fluorine bond.

As used herein, the term "**perfluorinated**" and "**perfluorocarbon**" refers to chemical compounds that are analogs of hydrocarbons wherein all hydrogen atoms in the hydrocarbon are replaced with fluorine atoms. Perfluorinated molecules can also contain a number of other atoms, including bromine, chlorine, and oxygen. A bromine substituted perfluorocarbon is a perfluorocarbon wherein one or more of the fluorine atoms have been replaced with a bromine atom. A chlorine substituted perfluorocarbon is a perfluorocarbon wherein one or more of the fluorine atoms have been replaced with a chlorine atom. A chlorine and bromine substituted perfluorocarbon is a perfluorocarbon wherein one or more of the fluorine atoms have been replaced with a chlorine atom and wherein one or more of the fluorine atoms have been replaced with a bromine atom. Examples of perfluorocarbons which may be suitable as a hydrophobic perfluorocarbonated moiety include those selected from perfluoroalkanes, perfluoroalkylamines, perfluoro-crown-ethers, perfluorinated alcohols, perfluorohaloalkanes, perfluorinated carboxylic acids, perfluorinated azides, perfluorinated thiols, perfluorinated alkenes, perfluorinated alkynes, perfluorinated acrylates, and perfluorinated esters.

Examples of hydrophobic perfluorinated moieties/groups may be selected from the group consisting of: perfluorinated substituted $C_1$-$C_{30}$ alkyl groups, perfluorinated unsubstituted $C_1$-$C_{30}$ alkyl groups, perfluorinated substituted alkoxy groups, perfluorinated unsubstituted alkoxy groups.

As used herein, the term "**group**" may refer to a functional group of a chemical compound. For the purpose of the present disclosure, the terms "**group**" and "**moiety**" may be used interchangeably. Groups of the present compounds refer to an atom or a collection of atoms that are a part of the compound. Groups of the present invention may be attached to other atoms of the compound via one or more covalent bonds. Groups may also be characterized with respect to their valence state. The present term may thus also include groups characterized as monovalent, divalent, trivalent, etc. valence states.

As used herein, the term "**substituted**" refers to a compound wherein a hydrogen is replaced by another functional group.

As used herein, the term "**polymer**" refers to a molecule comprising a plurality of repeating chemical groups, typically referred to as monomers.

As used herein, the term "**copolymer**", also commonly referred to as a heteropolymer, is a polymer formed when two or more different types of monomers are linked in the same polymer.

As used herein, the term "**block copolymer**" refers to a type of copolymer comprising blocks or spatially segregated domains, wherein different domains comprise different polymerized monomers. In a block copolymer, adjacent blocks are constitutionally different, i.e. adjacent blocks comprise constitutional units derived from different species of monomer or from the same species of monomer but with a different composition or sequence distribution of constitutional units. Different blocks (or domains) of a block copolymer may reside on different ends of a polymer (e.g. [A] [B]), or may be provided in a selected sequence ([A][B][A][B]).

As used herein, the term "**diblock copolymer**" refers to block copolymers having two different chemical blocks. "**triblock copolymer**" refers to block copolymers having three different chemical blocks.

Block copolymers may include block copolymers having a first block comprising a first polymer such as a PEG polymer, for example a PEG polymer having 8 to 300 monomers, a second polymer, an intermediate block such as a fluorocarbon, including but not limited to, a fluorocarbon such as a fluorinated or perfluorinated alkane, and a third interior hydrophobic block. Block copolymers of the present invention are capable of undergoing self-assembly to make supramolecular structures, such as encapsulated droplets.

As used herein, the term "block copolymer" may thus include compounds comprising at least a first block, said first block optionally comprising or consisting of a first polymer such as PEG polymer, and at least a second block, said second block optionally comprising or consisting of a second polymer. The term « block copolymer » may also include functionalized block copolymers, such as copolymers having additional moieties.

As used herein, an "**amphiphilic block copolymer**" may thus include compounds comprising at least one first block which is hydrophilic (e.g. a hydrophilic polymer such as a PEG polymer), and at least one second block which is hydrophobic. In the context of block copolymers, a hydrophilic block is more hydrophilic than a hydrophobic group of an amphiphilic block copolymer, and a hydrophobic group is more hydrophobic than a hydrophilic block of an amphiphilic polymer.

As used herein, the terms "**pharmaceutically acceptable salt**" or "**physiologically acceptable salt**" may refer to salts which are formed from acid addition salts formed with inorganic acids (e.g. hydrochloric acid, hydrobromic acid, sulfuric acid, phosphoric acid, nitric acid, and the like), as well as salts formed with organic acids such as acetic acid, oxalic acid, tartaric acid, succinic acid, malic acid, fumaric acid, maleic acid, ascorbic acid, benzoic acid, tannic acid, palmoic acid, alginic acid, polyglutamic acid, naphthalene sulfonic acid, naphthalene disulfonic acid, and polygalacturonic acid. Suitable physiologically acceptable acid addition salts of compounds, for example of amphiphilic molecules, include hydrobromide, tartrate, citrate, trifluoroacetate, ascorbate, hydrochloride, tosylate, triflate, maleate, mesylate, formate, acetate and fumarate.

As used herein, the term "**halogen**" may, in particular, refer to chlorine, fluorine, bromine, or iodine.

As used herein, the term "**alkyl**" may refer to linear or branched alkyl groups. In general alkyl groups include those having from 1 to 30 carbon atoms ; which may thus include those having from 1 to 2, 3, 4, 5, 6, 7, 8, 9, 10, 11, 12, 13, 14, 15, 16, 17, 18, 19, 20, 21, 22, 23, 24, 25, 26, 27, 28, 29 and 30 carbon atoms. For example $C_1$ to $C_x$ alkyl groups, also referred as ($C_1$-$C_x$) alkyls, may include ($C_1$-$C_2$) alkyls, ($C_1$-$C_3$) alkyls, ($C_1$-$C_4$) alkyls, ($C_1$-$C_5$) alkyls and ($C_1$-$C_6$) alkyls. Examples are, but are not limited to, methyl, ethyl, 1-propyl, 2-propyl, butyl, pentyl.

As used herein, the term "**substituted alkyl**" may include, among other alkyls, fully halogenated (e.g. perfluorinated) or semihalogenated alkyl groups, such as alkyl groups having one or more hydrogens replaced with one or more fluorine atoms, chlorine atoms, bromine atoms and/or iodine atoms. Substituted alkyl groups include fully fluorinated (i.e. perfluorinated) or semifluorinated alkyl groups, such as alkyl groups having one or more hydrogens replaced with one or more fluorine atoms. The term may also include, among other alkyls, those which are substituted with aryl groups, which in turn can be optionally substituted. Specific alkyl groups include methyl, ethyl, n-propyl, iso-propyl, cyclopropyl, n-butyl, s-butyl, t-butyl, cyclobutyl, n-pentyl, branched-pentyl, cyclopentyl, n-hexyl, branched hexyl, and cyclohexyl groups, all of which are optionally substituted.

As used herein, the term "**alkoxy**" may refer to an alkyl group (R) that has been modified by linkage to oxygen and can be represented by the formula (R-O) and can also be referred to as an alkyl ether group. Examples of alkoxy groups include, but are not limited to, methoxy, ethoxy, propoxy, butoxy and heptoxy. Alkoxy groups include substituted alkoxy groups wherein the alkyl portion of the groups is substituted as provided herein in connection with the description of alkyl groups.

As used herein the term "**carbonyl**" may refer to -(CO)-, wherein (CO) indicates that the oxygen is connected to the carbon with a double bond.

As used herein the term "**metal complexing moiety**" or "**metal complexing agent**" refers to any compound that contains one or more electron-donor atoms capable of forming coordinate bonds with a metal atoms ; and in particular metal atoms suitable for use as radioisotopes (i.e. radioactive metal ions), in particular in radiotherapy, such as zirconium-89, thorium-227, iodine-131, strontium-89, samarium-153, radium-223, radium-226, cesium-137, gold-198, tantalum-182, iridum-192. Some metal complexing moiety may, in particular, be chelating agents (e.g. desferrioxamine), namely compounds that contains two or more electron-donor atoms that are capable of forming coordinate bonds with a metal atom (e.g. a radioactive metal atom). Examples of metal complexing moieties according to the disclosure include desferrioxamine (also reported herein as deferoxamine or DFOA), ethylenediaminetetraacetic acid (EDTA), hydroxyethylethylenediaminetriacetic acid, nitrilotriacetic acid, N-dihydroxyethylglycine, ethylenebis(hydroxyphenylglycine), and dodecane tetraacetic acid (DOTA).

As used herein the term "**hydrophilic metal complexing moiety**" refers to a metal complexing moiety as defined above, which comprises at least one hydrophilic group.

As used herein, the terms "**treat**", "**treating**" and "**treatment**" are meant to include alleviating or abrogating a disorder, disease, or condition, or one or more of the symptoms associated with the disorder, disease, or condition; or alleviating or eradicating the cause(s) of the disorder, disease, or condition itself.

As used herein, the terms "**prevent**", "**preventing**", and "**prevention**" mean reducing the risk of onset or slowing the occurrence of a given phenomenon, namely in the present invention, a cancer and/or dysplasia and more particularly, a pre-cancerous condition, an early stage cancer or a non-metastatic cancer. The term "**preventing**" also encompasses **"reducing the likelihood of occurrence"** or **"reducing the likelihood of reoccurrence".**

As used herein, the term "**subject**" refers to an animal, including, but not limited to a human and non-human mammal, including a primate (e.g., human), cow, sheep, goat, horse, dog, cat, rabbit, rat, or mouse. The terms "subject" and "patient" are used interchangeably herein in reference, in particular, to a mammalian subject, such as a human.

As used herein, the terms "**tumor**" "**neoplasm**" and "**neoplastic disorder or disease**" are used interchangeably herein and are meant to refer to unwanted cell proliferation of one or more subset of cells in a multicellular organism resulting in harm (i.e., discomfort or decreased life expectancy) to the multicellular organisms. In certain embodiments, a tumor can be benign (non-invasive) or malignant (invasive).

As used herein, the term "**cancer**" is meant to refer to a malignant neoplasm, which is characterized by uncontrolled cell proliferation where cells have lost their normal regulatory controls that would otherwise govern the rate of cell growth. These unregulated, dividing cells can spread throughout the body and invade normal tissues in a process referred to as "metastasis". In the absence of other indications, the term is not construed to apply solely to one type of cancer, and may thus encompass those selected from: colorectal cancer, pancreatic cancer, lung cancer including non-small cell lung cancer, breast cancer, bladder cancer, gall bladder cancer, thyroid cancer, melanoma, liver cancer, uterine/cervical cancer, oesophageal cancer, kidney cancer, ovarian cancer, prostate cancer, head and neck cancer, and stomach cancer.

As used herein, the term "**contacting**" or "**contact**" is meant to refer to bringing together of a therapeutic agent and cell or tissue such that a physiological and/or chemical effect takes place as a result of such contact. Contacting can take place in vitro, ex vivo, or in vivo. In one embodiment, a therapeutic agent is contacted with a cell in cell culture

(in vitro) to determine the effect of the therapeutic agent on the cell. In another embodiment, the contacting of a therapeutic agent with a cell or tissue includes the administration of a therapeutic agent to a subject having the cell or tissue to be contacted.

As used herein, the term "**radiosensitization**" refers to the act of bringing into contact a cell or tissue, especially a tumor cell or tumor tissue, with a given compound or composition in order to render the said cell or tissue more sensitive to radiation therapy at a given dose of radiation. Accordingly, a radiosensitized cell or tissue may lead to a decreased survival fraction at the given dose, and/or an increased radiation enhancement ratio, and/or an increased sensitizer enhancement ratio.

As used herein, the term "**photodynamic therapy**" or "**PDT**" refers to the act of bringing into contact a cell or tissue, especially a tumor cell or tumor tissue, with a given compound or composition, which is then activated as a "photosensitizer" by light (e.g. a laser or any other source of light such as LEDS) at a certain wavelength, in order to produce reactive oxygen species and kill said cell or tissue. Hence, PDT can be distinguished as an alternative to radiotherapy, although both strategies can be complementary.

As used herein, the term "**survival fraction**" or "**SF**" refer to the fraction of a population of cells in culture (in particular tumor cells) which remain alive, at a given dose of radiation, compared to the original population of cells. For example, the corresponding survival fraction at a dose of 2 Gy may be reported as $\mathbf{SF_{2Gy}}$.

As used herein, the terms "**radiation enhancement ratio**" and " **RER**" are used interchangeably herein and refer to the ratio of survival fractions, at a given dose, without (control) and with a specific treatment.

As used herein, the term "**sensitizer enhancement ratio**" and "**SER**" refer to the ratio of the radiation dose required to obtain a surviving fraction (SF) of 0.5, under a control condition (e.g. without actual treatment), to that required to obtain the same SF after treatment. For example, the radiation dose required to obtain a SF of 0.5 under control condition is reported as $\mathbf{MID_{control}}$. For example, the radiation dose required to obtain a SF of 0.5 after treatment with PFTD-PEG micelles according to the present disclosure, is reported as $\mathbf{MID_{PFTD\text{-}PEG}}$.

As used herein, the terms "**dose enhancement ratio**" and "**dose enhancement factor**" and "**DEF**" are used interchangeably herein and are used to measure the fold increase in cytotoxic efficacy of radiation when delivered in the presence of a radiosensitizer. For example, the corresponding radiation may correspond to a dose of 2 Gy, in which case the corresponding DEF can be reported $\mathbf{DEF_{2Gy}}$. In certain embodiments, dose enhancement ratio is calculated by dividing the radiation dose required to achieve a surviving fraction (SF) in the absence of the radiosensitizer with the radiation dose required to achieve the same surviving fraction (SF) in the presence of the radiosensitizer at a specified concentration. This calculation method can be used to assess the radiosensitizing effect of a radiosensitizer in an in vitro system, such as cells in culture. In certain embodiments, the dose enhancement ratio is calculated by dividing normalized growth delay in subject(s), such as mice, treated with a radiosensitizer and radiation by absolute tumor growth delay in subject(s) of the same species but treated with radiation alone, wherein the absolute tumor growth delay in subject(s) treated with radiation alone is defined as the difference between the time in days for tumor(s) in the subject(s) treated with radiation to grow from a first specified size to a second specified size, such as from 7 mm to 12 mm for mice, and the time in days for tumor(s) in untreated subject(s) of the same species to grow from the first specified size to the second specified size; and normalized growth delay in subject(s) treated with a radiosensitizer and radiation is defined as the difference between the time in days for tumor(s) in subject(s) treated with both the radiosensitizer and radiation to grow from the first specified size to the second specified size and the time in days for tumor(s) in subject(s) of the same species treated with the radiosensitizer alone to grow from the first specified size to the second specified size.

## EXAMPLES

### Material & Methods

### 1. Chemical synthesis and micelle assembly

Two different perfluorinated amphiphiles were synthesized: one intended to optimize biocompatibility of the micelles and the other to permit the complexation of radioactive zirconium and thus allow for positron emission tomography (PET). The first amphiphile, referred to as PFTD-PEG (**1**), consists of a hydrophobic fluorinated moiety, derived from perfluorotetradecanol (PFTD) attached to a polyethylene glycol (PEG) chain, which serves as biocompatible hydrophilic headgroup. The second amphiphile, referred to as PFTD-DFO (**2**), is based on the same PFTD fluorinated region, but this time associated to a metal complexing desferrioxamine (DFO) derivative as the headgroup. The synthesis of PFTD-PEG and PFTD-DFO are summarized in **figures 1** and **2**, along with the synthesis of intermediate compounds **5, 6**, and **7**; as detailed hereafter.

*1.1 Synthesis of compound **4*** (or "*tosylated PEG*")

Under $N_2$ atmosphere, to a solution of PEG-2000 (2 g, 1 mmol, 1 equiv.) in dry $CH_2Cl_2$ (20 mL) was added $NEt_3$ (0.7 mL, 5 mmol, 5 equiv.). The reaction mixture was cooled to 0 °C before the portion-wise addition of p-tosyl chloride (953 mg, 5 mmol, 5 equiv.). The reaction was warmed up to room temperature overnight and monitored by TLC ($CH_2Cl_2$/methanol 90:10). Once complete, reaction mixture was washed sequentially with water, saturated $NH_4Cl$ solution, saturated $NaHCO_3$ solution and brine. The organic layer was dried over $MgSO_4$, filtered, and concentrated *in vacuo.* Purification was carried out by precipitation of the product in $Et_2O$ at -20 °C for 2 h. Compound **4** (1.9 g, 88%) was obtained as a white solid.

*1.2 Synthesis of PFTD-PEG (**1**)*

Under $N_2$ atmosphere, to a solution of perfluorotetradecanol **3** (2.46 g, 3.52 mmol, 4 equiv.) in dry THF (80 mL) was added NaH (135 mg, 60% in oil, 3.52 mmol, 4 equiv.). The reaction mixture was heated to 75 °C for 30 minutes to allow activation of the perfluorinated alcohol before addition of compound **4** (1.9 g, 0.88 mmol, 1 equiv.) dissolved in dry THF (10 mL). The reaction mixture was stirred for 3 days at 75 °C. Completion of the reaction was monitored by TLC (cylcohexane/EtOAc 70:30). Once complete, the reaction mixture was cooled to room temperature and concentrated *in vacuo.* The crude product was dissolved in $CH_2Cl_2$ and was filtered through Celite® to remove perfluorotetradecanol **3**. The resulting filtrate was concentrated under reduced pressure. PFTD-PEG (**1**) (1.5 g, 63%) was obtained as a white solid by precipitation in $Et_2O$ at -20 °C for 2 h. The synthesis of PFTD-PEG from perfluorotetradecanol **3** and tosylated PEG (MW = 2000) is as illustrated in **figure 1**.

*1.3 Assembly of PFTD-PEG micelles and DLS*

PFTD-PEG (**1**) (20 mg) was suspended in ultrapure water (2 mL) and sonicated with an ultrasonic probe (300 ms pulse per second, output 45%, Branson 550 Sonifier) for 10 min leading to a final micelle concentration of 10 mg $mL^{-1}$. The size of the PFTD-PEG micelles was measured by dynamic light scattering (Cordouan Vasco-Flex, equipped with a 450 nm laser, at 25 °C) which indicated a mean hydrodynamic diameter of 12 nm.

*1.4 Determination of Critical Micelle Concentration (CMC)*

The CMC was evaluated by measuring the surface tension of micelle suspensions with a tensiometer (Kibron EZ-Pi+, Kibron Inc.). Solutions at different concentrations of amphiphilic units (from 0.01 to 1 mg $mL^{-1}$) were prepared in water and the surface tension was measured (in triplicate), using the du Noüy-Padday method. The surface tension was plotted as a function of the concentration. The CMC value was obtained at the intersection of the two lines and was found to be 0.055 mg $mL^{-1}$.

*1.5 Synthesis of compound **5***

To a solution of tetraethylene glycol (22 g, 113 mmol, 10.3 equiv.) in THF (4 mL, 0 °C) was added a solution of sodium hydroxide (0.7 g, 17.4 mmol, 1.6 equiv.) in $H_2O$ (4 mL). A solution of p-tosyl chloride (2.08 g, 10.9 mmol, 1 equiv.) in THF (4 mL) was slowly added using a syringe pump over a period of 2 h. After completion of the p-tosyl chloride addition, reaction was carried out for another hour at 0 °C. The mixture was added to iced water (100 mL) and extracted with $CH_2Cl_2$ ($\times$ 3). The combined organic layers were washed with $H_2O$ then dried over $MgSO_4$. The solvent was removed under reduced pressure to afford the mono-activated tetraethylene glycol derivative **5a** as a slightly yellow oil (3.58 g, 94%). $Ph_3CSH$ (3.55 g, 12.84 mmol, 1.25 equiv.) was dissolved in a solution of EtOH/toluene 1:1 (17 mL) and NaOH (0.514 g, 12.84 mmol, 1.25 equiv.) in $H_2O$ (7 mL) was added to the reaction mixture. The mono-activated tetraethylene glycol derivative (3.58 g, 10.3 mmol, 1 equiv.) was then added to the medium and the resulting reaction mixture was stirred overnight at room temperature. Completion of the reaction was monitored by TLC (cyclohexane/EtOAc 20:80) and, once complete, the reaction mixture was poured into a saturated solution of $NaHCO_3$ and washed three times. Combined organic layers were washed with brine, dried over $MgSO_4$, filtered, and concentrated *in vacuo.* The crude product was purified by flash chromatography on silica gel eluted with cyclohexane/EtOAc from 80:20 to 20:80, yielding compound **5** as a yellowish oil (3.5 g, 76%).

*1.6 Synthesis of compound **6***

Under $N_2$ atmosphere, to a solution of compound **5** (2.62 g, 5.8 mmol, 1 equiv.) in dry $CH_2Cl_2$ (100 mL) was added $NEt_3$ (4 mL, 29 mmol, 5 equiv.). The reaction medium was cooled to 0 °C and p-tosyl chloride (2.21 g, 11.6 mmol,

2 equiv.) was added portion-wise while stirring. The reaction was warmed up overnight to room temperature and monitored by TLC (cyclohexane/EtOAc 70:30). Once complete, the reaction mixture was washed with $H_2O$ (× 2) and brine. The organic layer was dried over $MgSO_4$, filtered, and concentrated under vacuum. Purification of the crude product by flash chromatography on silica gel eluted with cyclohexane/EtOAc from 90:10 to 70:30 provided the tosylate **6a** as a transparent oil (2.8 g, 80%)

Under $N_2$ atmosphere, to a solution of perfluorotetradecanol **3** (12.8 g, 18.2 mmol, 4 equiv.) in dry THF (100 mL) was added NaH (698 mg, 18.2 mmol, 4 equiv.). The reaction mixture was heated to 75 °C for 30 min to allow activation of the perfluorinated alcohol before addition of the tosylate (2.8 g, 4.6 mmol, 1 equiv.) dissolved in dry THF (10 mL). Reaction mixture was stirred for 3 days at 75 °C. Once complete, the reaction mixture was cooled to room temperature and concentrated *in vacuo.* The crude product was dissolved in $CH_2Cl_2$ and was filtered through Celite® to remove perfluorotetradecanol **3**. The resulting filtrate was concentrated under reduced pressure. The crude product was purified by flash chromatography on silica gel eluted with cyclohexane/EtOAc from 98:2 to 80:20. The desired perfluorinated thiol-protected product **6b** was recovered as a white solid (1.93 g, 61%).

Under $N_2$ atmosphere, to a solution of perfluorinated thiol-protected compound (600 mg, 0.53 mmol, 1 equiv.) in dry $CH_2Cl_2$ (20 mL) was added TIPS (0.6 mL, 2.64 mmol, 5 equiv.). The reaction mixture was allowed to cool to 0 °C for 30 min before addition of TFA (0.2 mL, 2.64 mmol, 5 equiv.). The reaction mixture was warmed up overnight to room temperature and the reaction was monitored by TLC (cyclohexane/EtOAc 80:20). Once TLC indicated complete consumption of the starting material, the reaction mixture was concentrated *in vacuo,* adding acetone to the medium to facilitate TFA evaporation. The crude product was purified by column chromatography on silica gel eluted with cyclohexane/EtOAc 2:1 to give compound **6** as a white solid (360 mg, 72%).

*1.7 Synthesis of iodoacetic acid N-hydroxysuccinimide ester*

Under $N_2$ atmosphere, to a solution of iodoacetic acid (372 mg, 2 mmol, 1 equiv.) in dry $CH_2Cl_2$ (20 mL) was added dicyclocarbodiimide (DCC, 412 mg, 2 mmol, 1 equiv.). The reaction mixture was cooled to 0 °C and NHS (230 mg, 2 mmol, 1 equiv.) was added portion-wise. The reaction was warmed up to room temperature and was stirred for 4 h before completion. Precipitated DCU was filtered off and the obtained filtrate was concentrated *in vacuo.* Recrystallization in isopropanol and filtration yielded N-hydroxysuccinimidyl iodoacetate as a white solid (430 mg, 76%).

*1.8 Synthesis of compound 7*

Desferrioxamine mesylate (200 mg, 0.30 mmol, 1 equiv.) and N,N-diisopropylethylamine (53 μL, 0.30 mmol, 1 equiv.) were mixed in DMF (4 mL) and water (0.4 mL). *N*-hydroxysuccinimidyl iodoacetate (93 mg, 0.33 mmol, 1.1 equiv.) was added to the mixture and the reaction was stirred at room temperature for 2 h. Water (8 mL) was added and the precipitated product was filtered off, washed with water and dried under reduced pressure. Compound **7** (129 mg, 59%) was obtained as a white powder.

*1.9 Synthesis of PFTD-DFO (2)*

Under $N_2$ atmosphere, to a solution of compound **7** (20 mg, 0.027 mmol, 1 equiv.) and compound **6** (25 mg, 0.027 mmol, 1 equiv.) in dry DMSO (4 mL) was added $K_2CO_3$ (4 mg, 0.027 mmol, 1 equiv.). The reaction mixture was stirred at room temperature for 24 h, poured in water and freeze-dried. Several washing steps with ultrapure water and EtOAc followed by centrifugation allowed the obtention of pure PFTD-DFO (**2**) as a white solid (14 mg, 35%). The synthesis of PFTD-DFO is as illustrated in **figure 2.**

*1.10 Assembly of PFTD-PEG (**1**) /PFTD-DFO (**2**) 90:10* w/w *micelles and DLS*

PFTD-PEG (**1**) (13,5 mg) and PFTD-DFO (1,5 mg) were solubilized in a 0.1 M NaOH solution (1 mL) and sonicated with an ultrasonic probe (300 ms pulse per second, output 45%, Branson 550 Sonifier) for 10 min leading to a final micelle concentration of 15 mg $mL^{-1}$. Buffer exchange using NAP10 yielded micellar PFTD-PEG (**1**) /PFTD-DFO (**2**) 90:10 w/w solution at 10 mg $mL^{-1}$. The size of PFTD-PEG (**1**) /PFTD-DFO (**2**) 90:10 w/w micelles was measured by dynamic light scattering (Cordouan, Vasco-Flex, equipped with a 450 nm laser at 20 °C), which indicated a mean hydrodynamic diameter of 15 nm.

*1.11 Determination of zeta potential of perfluorinated micellar solutions*

Zeta potentials of the different micellar formulations were obtained with a Wallis Zeta potential analyser at 20 °C.

## *2. In vitro* evaluation

### *2.1. Cellular experiments*

B16F10 cells (ATCC®, CCL-6475™), were cultured in Dulbecco's Modified Eagle Medium with Glutamax (Gibco) supplemented with 10% fetal bovine serum (Gibco) and 1% penicillin-streptomycin (Gibco, 10 000 U $mL^{-1}$). Cells were grown in a humidified incubator at 37 °C and 5% $CO_2$.

### *2.2 Study of internalization of perfluorinated micelles by confocal microscopy*

B16F10 cells were seeded on 35 mm glass dishes in a 6-well plate at a cell density of 1.5 $10^3$ cells/well in DMEM with 10% FBS and 1% antibiotic/antimycotic solution and left to adhere for 24 h in a humidified 5% $CO_2$ atmosphere at 37 °C. After 24 h, cells were incubated with fluorescent perfluorinated micelles (c = 0.5 mg $mL^{-1}$, loaded with 1 wt% DiD) in DMEM supplemented with 10% FBS and 1% antibiotic/antimycotic solution for 24 h. After exposure, cells were washed with DPBS 1× (× 3), fixed in 4% paraformaldehyde for 20 min, stained with 1 μM Hoechst 33342 for 20 min then washed with DPBS 1× and observed by confocal microscopy. DiD was excited at 644 nm and the fluorescence emission was detected in the 650-750 nm range. A confocal laser scanning microscope (LEICA SP5) was used to directly visualize the intracellular location of perfluorinated micelles.

### *2.3 In vitro cellular uptake study of perfluorinated micelles by flow cytometry*

B16F10 cells were seeded in 6-well plates at different cellular densities in order to reach 80-90% confluence during the time of the experiment, ranging from 7.5 $10^4$ cells $mL^{-1}$ to 4.5 $10^5$ cells $mL^{-1}$ in DMEM with 10% FBS and 1% antibiotic/antimycotic solution and left to adhere for 24 h in a humidified 5% $CO_2$ atmosphere. After 24 h, cells were incubated with perfluorinated micelles loaded with 1 wt% DiD. After different times, the medium was discarded, cells were washed twice with DPBS 1×, trypsin (500 μL) was added to the well and cells were incubated for 5 min at 37 °C. Trypsin was inhibited by addition of DMEM supplemented with 10% FBS (3 mL) and cells were transferred into flow cytometry tubes and centrifuged (1000 ×g, 5 min) before being resuspended in cold DPBS (200 μL). Cells were kept on ice until measurement. Fluorescence intensity of 10 000 cells was recorded by flow cytometry and a mean value was calculated. For each condition, mean fluorescence values were corrected by subtraction of autofluorescence.

### *2.4 In vitro endocytosis pathway study by flow cytometry*

24 h prior to the experiment, B16F10 cells were seeded in a 6-well plate at a density of 4.5 $10^5$ cells $mL^{-1}$ (2 mL per well) in DMEM with 10% FBS and 1% antibiotic/antimycotic solution and left to adhere in a humidified 5% $CO_2$ atmosphere. Cells were then incubated either with 800 μM amiloride, 400 μM genistein or 0.45 M sucrose for 30 min. After 30 min of pretreatment, perfluorinated micelles loaded with 1 wt% DiD were added to the medium and cells were incubated for another 2 h in the presence of micelles. To investigate the influence of the temperature, cells were kept at 4 °C for 2.5 h. After treatment, the medium was discarded, cells were washed with DPBS 1× (× 3) and detached by trypsinization (500 μL). Trypsin was neutralized by addition of DMEM supplemented with 10% FBS (3 mL) and cells were transferred into flow cytometry tubes before being centrifuged (1000 ×g, 5 min) and resuspended in cold DPBS 1×. Cells were kept on ice before measurement. Fluorescence intensity of 10 000 cells was recorded by flow cytometry and a mean value was calculated. For each condition, mean fluorescence values were corrected by subtraction of autofluorescence and normalized, control experiment (no inhibitor) taken as the reference (100%).

### *2.5 Cytotoxicity study of perfluorinated micelles by MTS assay*

B16F10 cells were plated into a 96-well plate at a density of 8 $10^3$ cells per well in medium (100 μL) and left to adhere for 24 h in a humidified 5% $CO_2$ atmosphere. After plating, B16F10 cells were incubated with different concentrations of perfluorinated micelles, ranging from 0.1 to 1 mg $mL^{-1}$, for 24 h after which MTS reagent (20 μL) was added to each well. After incubation at 37 °C for 2 h, the plate was read on a microplate reader at 490 nm. The viability change of micelle-treated cells was expressed as a percentage compared to the cells without micelle treatment, and control cells were considered to have a viability value of 100%. Each experiment was conducted in triplicate.

### *2.6 Clonogenic survival assay*

B16F10 cells were plated in a T25 flask at a density of 2 $10^5$ cells $mL^{-1}$ and left to adhere for 24 h at 37 °C in a humidified 5% $CO_2$ atmosphere. After plating, the medium was discarded and cells were incubated for 24 h with

different concentrations of perfluorinated micelles, ranging from 0.1 mg $mL^{-1}$ to 1 mg $mL^{-1}$. After treatment, the micelle-containing medium was discarded, cells were washed with DPBS 1×, detached by trypsinization (500 μL) and counted. B16F10 cells were then seeded in 10 cm Petri dishes and kept for 7 days in a humidified incubator at 37 °C at a density leading to approximatively 100 colonies. Subsequently, cells were stained with 0.5% crystal violet and colonies were counted. A colony was defined as a clone containing at least 50 cells.

*2.7 In vitro radiosentization assay*

B16F10 cells were plated in T25 flasks at different seeding densities, from 100 to 2800 cells per flask, and left to adhere for 24 h in culture medium at 37 °C in a humidified 5% $CO_2$ atmosphere. The amount of cells seeded was adapted to each irradiation dose, in order to have between 50 and 100 colonies formed, even at higher irradiation doses. After attachment, the cells were incubated for 24 h in culture medium with or without perfluorinated micelles (0.25 mg $mL^{-1}$). 3 flasks per tested condition were prepared according to the presence of micelles or not and to the irradiation dose. After treatment with micelles, the medium was discarded and replaced by fresh medium and cells were irradiated with a $^{137}Cs$ sealed source at 1 Gy $min^{-1}$ (GSR-D1 gamma irradiator, $^{137}Cs$) for different time periods resulting in doses ranging from 0 to 6 Gy. After irradiation, cells were kept at 37 °C in a humidified incubator for 7 days. Subsequently, cells were stained with 0.5% crystal violet and the resulting colonies (> 50 cells) were counted. The plating efficiency (PE), calculated relative to the control group (0 Gy), was defined as the ratio of the number of colonies formed to the number of cells seeded. Surviving fraction (SF) was calculated as follows: SF = (# of colonies formed) / (# of cells seeded × *PE*).

*2.8 Statistical analysis*

Statistical data analysis was performed using GraphPad Prism (v9.1.2, GraphPad Software). For flow cytometry experiments, data are shown as mean ± *SEM* whereas for irradiation experiments and toxicity experiments, data are shown as mean ± *SD*. All experiments were carried out in triplicate. Student's t test or one-way ANOVA analysis of variance was utilized to determine the statistical significance between two or more groups. The significance of difference was indicated as $p < 0.05$ (*$p < 0.05$, **$p < 0.01$, ***$p < 0.001$, ****$p < 0.0001$). For irradiation experiments, data were fitted using OriginPro second order polynomial fit.

**3. *In vivo* and *Ex vivo* studies**

*3.1. Animal models*

Animal experiments were performed according to the European Directive 2010/63/EU and to its transposition into the French law (Decree No. 2013-118). The research project was conducted at the CEA-SHFJ imaging platform and was approved by a local ethics committee. Animals were housed in individually-ventilated cages with free access to food and water in a temperature- and humidity-controlled room (22 °C, 40%), with a 12-h light/12-h dark cycle. Animal experiments were performed under anaesthesia with isoflurane in oxygen. 5 week old female C57BL/6 mice were purchased from Janvier laboratory. For tumor imaging studies, mice were subcutaneously injected with 1 $10^6$ B16F10 cells suspended in DPBS (1× 100 μL) into both flanks while anesthetized with 2% isoflurane.

*3.2 Tolerance study*

Three healthy 5 week old C57BL/6 were intravenously injected through the vein tail with PFTD-PEG micellar solution in 0.5 M HEPES (100 μL, $c_{stock}$ = 10 mg $mL^{-1}$) and monitored for 5 days.

*3.3 Radiolabeling of perfluorinated micelles containing 10% PFTD-DFO amphiphile*

[$^{89}$Zr]Zr-oxalate (85 μL, 3.1 mCi) was neutralized by 2 M $Na_2CO_3$ (38 μL) in an Eppendorf tube. The tube was gently shaken and degassed then 90:10 PFTD-PEG/PFTD-DFO w/w micellar solution in 1 M HEPES (800 μL, 10 mg $mL^{-1}$, pH 7.1-7.3) was added to the mixture. The reaction was incubated at 37 °C under orbital shaking for 1 h and radiolabeling efficiency was determined by iTLC. Further purification on PD10 column (GE Healthcare) using DPBS as the elution buffer yielded 100% radiolabeled 90:10 PFTD-PEG/PFTD-DFO w/w micelles in DPBS (2.84 mCi, 2.5 mL).

*3.4 Instant thin layer chromatography*

To assess the radiolabeling efficiency, a reaction sample (5 μL) was taken after 1 h incubation at 37 °C,

deposited on iTLC-SG glass microfiber papers impregnated with silica gel (Agilent Technologies) and eluted with citrate sodium buffer (20 mM, pH 5). The activity on the paper was detected using a Mini-Scan TLC Imaging Scanner (Eckert & Ziegler, Germany) at 1.0 mm $s^{-1}$ speed equipped with gamma detector B-FC-3600.

*3.5 Biodistribution study*

Subcutaneous B16F10 tumor bearing C57BL/6 mice (5 ± 1 weeks, 17.5 ± 1.4 g) were intravenously injected with the $^{89}$Zr-labeled perfluorinated micelles (4.8 ± 0.2 MBq). PET emission scans were performed using an Inveon microPET scanner and an Inveon microPET/CT scanner (Siemens). A 60-min dynamic PET scan was performed immediately after micelle injection. 20-min static PET scans were subsequently acquired at selected times (4 h, 24 h, 48 h, 72 h and 7 days) post-injection (p.i.). After each PET scan, a transmission scan or a CT scan was performed for photon attenuation correction. PET images were reconstructed with the Inveon Acquisition Workspace software (v2.1) using the OP-OSEM3D-MAP algorithm. Normalization and corrections for dead-time, scatter, decay and attenuation were applied to all PET data. Volumes of interest (VOIs) were defined with the PMOD software (v3.9). Fixed-size spherical VOIs (~ 8 $mm^3$) were drawn in representative parts of the heart, liver, kidney, spleen, muscle and brain. Bones and subcutaneous tumors were delineated semi-automatically. The mean activity concentration (kBq $cm^{-3}$) in each VOI was divided by the total injected dose (kBq) to obtain the percentage of injected dose per volume of tissue (%ID $cm^{-3}$).

*3.6 Ex vivo biodistribution*

After the last PET imaging session, mice were sacrificed by cervical dislocation. Blood and major organs (brain, heart, liver, kidney, spleen, intestine, tumor, muscle, bone) were collected, weighed, and counted with a Wizard2 gamma counter (PerkinElmer). Radioactivity data (in kBq) were background-corrected, decay-corrected to injection time and divided by the total injected activity (in kBq) and the organ weight (in g), to obtain the percentage injected dose per gram of tissue (%ID $g^{-1}$).

*3.7 Immunohistochemistry*

At 24 h and 48 h p.i., tumors were resected and snap-frozen. Excised tumors were stored at -80 °C until processing. Frozen tumors were cryo-sectioned into 5-μm thick slices and the resulting tumor sections were stained with Harris hematoxylin (Sigma-Aldrich) and Eosin Y alcoholic (Sigma-Aldrich). H&E staining was performed on autoradiography slides and on adjacent tumor sections.

Transmitted light images of stained tumor sections were acquired with the Axio Observer 5 microscope (Zeiss) at 10× magnification. Image post-processing (stitching, white balance, global contrast adjustment) was performed with the ZEN software (v2.6, Zeiss).

*3.8 Autoradiography study*

Tumor slides were exposed to a storage phosphor screen in a cassette (Molecular Dynamics) for 24 h. The screen was read in an imager (Storm 860 Molecular Imager, Molecular Dynamics). The luminescence emitted from the screen corresponded directly to the radioactivity on the section. The images were acquired at 50 μm resolution.

**Example 1. Amphiphile synthesis and micelle assembly.**

The PFTD-PEG amphiphile **1** was synthesized according to our previously reported procedure from perfluorotetradecanol (**3**) and tosylated PEG (**4**, *MW* = 2 124.56 g $mol^{-1}$) as shown in **Figure 1**.

The synthesis of PFTD-DFO first required the introduction of a terminal thiol group on the perfluorotetradecanol moiety through the use of a polyethylene glycol spacer (**figure 2A**). This was achieved by activation of tetraethylene glycol into its monotosyl derivative, followed by substitution with triphenylmethanethiol to yield intermediate **5**. The latter's hydroxyl end group was then activated into its tosylated derivative which permitted the *O*-alkylation of **3**, before deprotection under acidic conditions to yield compound **6**. The latter was reacted with iodoacetylated DFO **7** (**figure 2B**) in the presence of potassium carbonate to yield the target PFTD-DFO amphiphile **2** (**figure 2C**).

To better understand the behavior of amphiphiles **1** and **2** in aqueous medium, their critical micelle concentration (CMC) was determined by surface tension measurements at gradually increasing concentrations. These experiments allowed to determine a CMC value of 0.056 mg $mL^{-1}$ for PFTD-PEG. Micelles were obtained by ultrasonic treatment of either PFTD-PEG alone or a mixture of PFTD-PEG/PFTD-DFO in a 9:1 ratio, keeping the total amphiphile concentration at 10 mg $mL^{-1}$ in water (well above the individual CMC of the molecules). This resulted in the self-assembly of the amphiphiles into spherical micelles of about 12 nm in diameter, as observed by dynamic light scattering analyses (DLS).

The zeta potential of the micelles was also measured in order to assess whether or not the introduction of 10% DFO at their surface would convey a negative charge to the neutral PEG surface. Measurements showed a potential of 0 mV, regardless of the presence of the complexing unit at the micelle surface.

**Example 2. The micelle compositions according to the present disclosure possess improved oxygen transport properties.**

In order to experimentally demonstrate the improved ability of PFTD-based micelles of the present disclosure to embark oxygen, a series of experiments were performed, aiming at assessing the amount of oxygen released from PFTD-PEG colloids. To this end, a small volume of oxygen-saturated micelle colloid was injected with variable PFTD-PEG concentrations into a large volume of degassed water and the release of oxygen was monitored by continuous electrochemical monitoring over time (**Figure 3A**). From this set of experiments, a clear correlation between the micelle concentration and the amount of released oxygen was observed. In fact, 15 min after addition of 200 μL of a 5, 10, or 20 mg $mL^{-1}$ PFTD-PEG colloid to 100 mL of degassed water, the oxygen concentration reached values of 2.9, 4.3, and 5.6 μM, respectively. As a reference, the oxygen concentration reached only 1.1 μM when the same volume of oxygen-saturated water was added instead of the micelle colloid. The effect of the fluorinated PFTD-PEG micelles on oxygen delivery was compared to that of micelles obtained from commonly used amphiphiles, namely sodium dodecyl sulfate (SDS) and Triton X100. Upon addition of 200 μL of a 7.6 mM colloid of either of these surfactants, the oxygen concentration was found to be 2.7 and 3.6 mM, for SDS and Triton X-100, respectively. The values obtained with the reference surfactants are significantly lower than those observed with PFTD-PEG micelles at the same 7.6 mM concentration (20 mg $mL^{-1}$) and even lower than those obtained with only 3.8 mM of PFTD-PEG (10 mg $mL^{-1}$), confirming the excellent oxygen transport properties of the perfluorinated micellar vectors.

Another commonly used method consists in measuring the rate of decrease of the oxygen concentration of a given micellar solution from $O_2$-saturation to equilibrium with ambient air. In the case of perfluorinated micelles, any observed difference in deoxygenation rate can be directly related the diffusion of $O_2$ from the perfluorinated core to the aqueous medium while oxygen dissolved in the saturated dispersant (*i.e.* water) escapes to the atmosphere.

Such a method is, for example, disclosed in Fan et al. (ACS Appl. Bio Mater., 2018, 1, 708). This method provides a reliable estimate of the oxygen dissolution potential exhibited by perfluorinated self-assemblies, as also supported by Pitarresi et al. (J. Fluor. Chem., 2008, 129, 1096).

To assess the dioxygen release capacity of PFTD-PEG micelles, colloids (4 mL, c = 3.8 mM and 7.6 mM) were pre-saturated with dioxygen under agitation in a sealed round-bottom flask. After saturation, agitation was stopped and the PFTD-PEG micellar suspension was left to equilibrate with ambient air. Evolution of dioxygen concentration in the medium over time was recorded using a dissolved dioxygen sensor.

A similar set up was used for control experiments (water, 3.8 mM Triton X 100, 9 mM SDS). The results obtained are shown in Figure 3B and were fitted to a first-order exponential function model (Eq. 1), in agreement with the literature:

$$y = y_0 + Ae^{-x/t} \qquad (Eq.\ 1)$$

In this equation, yo corresponds to the value of y at t = 0 s while A corresponds to the amplitude of the curve such that A = Plateau - yo. This model provides essential parameters such as the oxygen half-life and the oxygen release rate from the different micellar suspensions. These parameters are listed in **Table 1**:

**Table 1: Parameters associated with oxygen release from different aqueous formulations.**

| **Formulation** | **$t_{1/2}$ (s)** | **Release rate ($10^{-4}$ $s^{-1}$)** |
|---|---|---|
| Water | 2316.1 ± 33.7 | 2.99 ± 0.04 |
| SDS (9 mM) | 2657.0 ± 29.9 | 2.61 ± 0.03 |
| Triton X-100 (3.8 mM) | 2785.3 ± 49.9 | 2.49 ± 0.04 |
| **PFTD-PEG** (3.8 **mM)** | **4106.8 ± 101.2** | **1.69 ± 0.04** |

A higher oxygen half-life was observed in the PFTD-PEG micellar suspension (10 mg $mL^{-1}$) compared to the other three conditions (water, 3.8 mM triton X 100, 9 mM SDS), reaching a value close to twice that obtained for water (4106 s for PFTD-PEG *vs* 2316 s for water). The oxygen release rate from the PFTD-PEG micellar suspension is also much slower than it is from the other formulations tested, suggesting that a significant amount of oxygen is retained within the micelles, diffusing only when the dissolved oxygen in the aqueous phase is in equilibrium with the oxygen in

the air. It thus appears that the $[O_2]_{micelle} \leftrightarrow [O_2]_{water}$ equilibrium offsets the $[O_2]_{water} \leftrightarrow [O_2]_{atm}$ equilibrium.

**Example 3. Dye encapsulation and cellular uptake in cancer cells.**

The interaction of PFTD-PEG micelles with cancer cells was studied by confocal microscopy. To this end, micelles were loaded with 1 wt% DiD, a hydrophobic organic dye with maximum emission wavelength of 665 nm. The fluorescent DiD@PFTD-PEG micelles were then incubated with B16F10 murine melanoma cells for 24 h, following which cell nuclei were stained with Hoechst 33342 dye and microscopic examination was carried out. Micrographs showed fluorescent spots densely distributed within the cytosol of the cells, most likely corresponding to endosomes/lysosomes. UV and DLS profiles are provided respectively in **Figure 8A** and **8B.**

No morphological alteration of the cells was observed, providing first evidence that the micelles are well-tolerated. It is also worth mentioning that DiD can be seen as a model hydrophobic compound, mimicking actual drug molecules that could be encapsulated inside the micelles and further transported and delivered to the intracellular compartment.

To better understand the mechanism involved in the uptake of PFTD-PEG micelles into B16F10 cells, fluorescence-assisted cell sorting (FACS) experiments were undertaken.

To elucidate their internalization pathway, DiD@PFTD-PEG micelles were incubated with B16F10 cells under inhibitory conditions. First, the unspecific inhibition of active internalization mechanisms was tested by incubation at 4 °C. Under such conditions, almost no internalization was observed, confirming that the objects entered cells by an active pathway. Then, specific chemical inhibitors were added to the culture medium, in separate experiments: i) amiloride (inhibitor of micropinocytosis); ii) sucrose (inhibitor of clathrin-mediated endocytosis); iii) genistein (inhibitor of caveolae-mediated endocytosis). Each experiment was analyzed by fluorescence-assisted cell sorting (FACS) in order to quantify the corresponding cellular uptake (**Figure 4**).

Through this set of experiments, it appeared that PFTD-PEG micelles were mainly internalized by caveolae- and clathrin-dependant pathways, while micropinocytosis was shown to contribute to the process, but to a lesser extent.

**Example 4. cytotoxicity assessment and radiosensitization of cancer cells.**

The toxicity of the micellar carriers was evaluated on B16F10 cultures with respect to both cell survival (MTS test) and colony forming ability (clonogenic assay).

For the MTS assay, cells were incubated for 24 h with PFTD-PEG micelles at concentrations ranging from 0.01 to 1 mg $mL^{-1}$. Phosphate buffer saline and 1 $\mu$M staurosporine were used as negative and positive controls, respectively. The amount of surviving cells was measured by colorimetric assay based on the conversion of a tetrazolium salt (MTS) into purple formazan by NAD(P)H-dependent cellular oxidoreductase enzymes. PFTD-PEG micelles were found to be well-tolerated, with no significant effect on cell survival, at concentrations ranging from 0.01 to 0.5 mg $mL^{-1}$. However, a slight toxicity was observed when cells were incubated with a micelle concentration of 1 mg $mL^{-1}$ (**Figure 5a**).

The ability of B16F10 cells to form colonies in the presence of PFTD-PEG micelles was also evaluated. To this end, 100 cells were seeded in Petri dishes and exposed to increasing concentrations of micelles for 24 h. The micelle-containing medium was then replaced with fresh culture medium and the culture was incubated for 24 h. The number of colonies was then counted to evaluate the impact of the micelles. As with the previous assay, no effect of the micelles was observed for concentrations up to 0.5 mg $mL^{-1}$, but mild toxicity was detected at 1 mg $mL^{-1}$ (**Figure 5b**).

In order to probe their radiosensitizing effect, PFTD-PEG micelles (0.25 mg $mL^{-1}$) were incubated with B16F10 cells for 24 h before irradiation at variable doses (from 0 to 6 Gy) using a $^{137}Cs$ sealed source. After irradiation, cell cultures were left in a humidified incubator for 7 days and a surviving fraction was measured based on the number of colonies were counted. The results (Table 2) were compared to those of a control experiment in which cells were incubated with culture medium containing no micelles prior to irradiation (**Figure 6**). A clear increase of the effect of irradiation was observed with a sensitizer enhancement ratio (*SER*) of 1.23 and a radiation enhancement ratio (*RER*) of 1.30 at 2 Gy, as illustrated in the following table, both of which compare favorably with other systems reported in the literature: Butterworth, K. T. et al., Nanoscale, 2012, 4, 4830; Subiel, A. et al., Theranostics, 2016, 6, 1651-1671.

**Table 2: perfluorinated micelles as efficient agents for radiosensitization**

| | SER | $SF_{2Gy}$ | $DMR_{20\%}$ | $DMR_{50\%}$ | $RER_{2Gy}$ |
|---|---|---|---|---|---|
| | $\frac{MID_{control}}{MID_{PFTD\text{-}PEG}}$ | SF at 2 Gy | $\frac{D_{contrôle\ 20\%}}{D_{PFTD\text{-}PEG20\%}}$ | $\frac{D_{control\ 50\%}}{D_{PFTD\text{-}PEG50\%}}$ | $\frac{SF_{xGy\ control}}{SF_{xGy\ PFTD\text{-}PEG}}$ |
| PFTD -PEG | **1.23** | 0.6774 | 1.1 | 1.24 | **1.3** |

(continued)

| | SER | $SF_{2Gy}$ | $DMR_{20\%}$ | $DMR_{50\%}$ | $RER_{2Gy}$ |
|---|---|---|---|---|---|
| | $\frac{MID_{control}}{MID_{PFTD\text{-}PEG}}$ | SF at 2 Gy | $\frac{D_{contrôle\ 20\%}}{D_{PFTD\text{-}PEG20\%}}$ | $\frac{D_{control\ 50\%}}{D_{PFTD\text{-}PEG50\%}}$ | $\frac{SF_{xGy\ control}}{SF_{xGy\ PFTD\text{-}PEG}}$ |
| Ctrl | - | 0.8813 | - | - | - |

With SER being the sensitizer enhancement ratio.

With RER being the radiation enhancement ratio.

This radiosensitization of the PFTD-PEG micelles can be attributed to their ability to carry large amounts of oxygen combined with their efficient cellular uptake.

This combination most likely results in a better transport of oxygen from the extracellular medium into cancer cells, leading to an increased amount of reactive oxygen species (ROS) formed within close vicinity of the cellular machinery along with the stabilization of direct DNA damage under irradiation, thus triggering higher cytotoxicity.

**Example 5. Tolerance and biodistribution in healthy mice and B16F10 tumors-bearing mice.**

Micelle colloids were injected intravenously to healthy mice that were then kept under monitoring (body weight, feeding behavior, tonicity) for 5 days. None of the treated animals showed any sign of sufferance over the period of the experiment, confirming that the micelles were well-tolerated.

Micelles containing 10 wt% of PFTD-DFO amphiphiles were first radiolabeled by incubation with $^{89}Zr$ oxalate followed by gel exclusion chromatography (PD10). Instant thin layer chromatography (iTLC) confirmed the efficient labeling (> 90%) of the micelles.

Radiolabeled micelles were then injected to mice bearing subcutaneous B16F10 tumors and the whole body distribution of the radioactive objects was monitored over 7 days. At that point, the animals were sacrificed and their organs collected for *ex vivo* analysis. Dynamic positron emission tomography (PET, **Figure 7**) showed liver accumulation building up within the first 48 h and then decreasing, suggesting hepatobiliary excretion of the micelles.

This was further supported by substantial radioactivity in the intestine whereas very little signal remained in the kidneys a few hours after injection.

Most interestingly, PET imaging demonstrated a significant accumulation of the micelles in the tumor, reaching a maximum of *ca.* 7% of the injected dose, 24 h post injection. The tumor/muscle ratio was also shown to be maximal (12.1 ± 2.3) between 24 and 48 h post injection. The PET-mediated biodistribution was confirmed by the *ex vivo* analysis of the isolated organs performed using gamma counting at day 7 and a solid correlation was established between the two sets of results ($R^2$ = 0.92).

## Claims

1. Biocompatible micelle composition, for use as a medicament, **characterized in that**:

   (i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons;
   (ii) the micelle has an average hydrodynamic diameter equal or inferior to 40 nm.

2. Biocompatible micelle composition for use according to claim 1, the composition being for use in radiotherapy.

3. Biocompatible micelle composition for use according to claim 1, the composition being for use in a method for treating or preventing tissue hypoxia in a patient.

4. Biocompatible micelle composition for use according to any of claims 1 to 3, the micelle having an average hydrodynamic diameter equal or inferior to 13 nm

5. Biocompatible micelle composition for use according to any of claim 1 to 4, the hydrophilic molecule comprising a polyethylene glycol (PEG) containing between 8 and 100 ethoxy monomers; in particular containing between 30

and 60 ethoxy monomers, and more particularly containing between 40 and 50 ethoxy monomers.

6. Biocompatible micelle composition for use according to any of claim 1 to 4, the plurality of amphiphilic molecules comprising or consisting of:

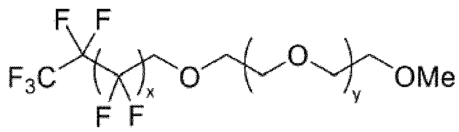

;

wherein **x** ranges from 8 to 15;
wherein **y** ranges from 8 to 100.

7. Biocompatible micelle composition for use according to any of claim 1 to 4, the plurality of amphiphilic molecules comprising or consisting of:

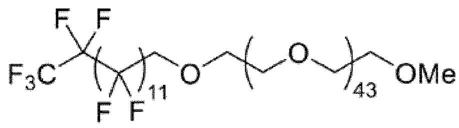

.

8. Biocompatible micelle composition for use according to any of the preceding claims, wherein the plurality of amphiphilic molecules comprises a hydrophobic perfluorinated moiety linked to a hydrophilic metal complexing moiety; in particular a hydrophilic metal complexing moiety comprising desferrioxamine or a derivative thereof.

9. Amphiphilic molecule, comprising at least one hydrophobic perfluorinated moiety linked to a hydrophilic metal complexing moiety; **characterized in that** the hydrophilic metal complexing moiety comprises desferrioxamine or a derivative thereof.

10. Amphiphilic molecule according to claim 9, consisting of:

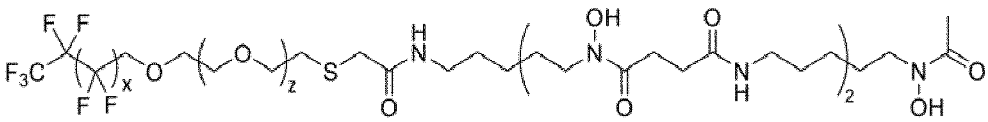

;

wherein **x** ranges from 8 to 15;
wherein z ranges from 0 to 10.

11. Amphiphilic molecule according to claim 10, consisting of:

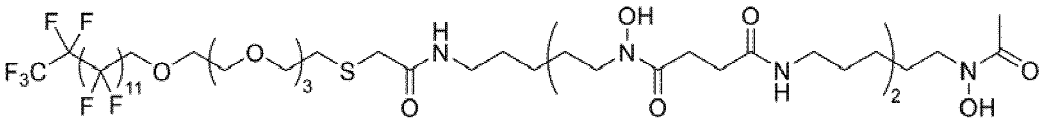

.

12. A method for preparing an amphiphilic molecule according to any of claims 9 to 11, comprising a step of bringing into contact a hydrophobic perfluorinated moiety with a hydrophilic metal complexing moiety comprising desferrioxamine or a derivative thereof.

13. Biocompatible micelle composition, the micelle comprising:

(i) a plurality of amphiphilic molecules comprising at least one hydrophobic perfluorinated moiety linked to at least one hydrophilic moiety;

(ii) a plurality of amphiphilic molecules comprising at least one hydrophobic perfluorinated moiety linked to at least one hydrophilic metal complexing moiety comprising desferrioxamine or a derivative thereof.

**14.** A kit comprising

(i) a plurality of amphiphilic molecules comprising at least one hydrophobic perfluorinated moiety linked to at least one hydrophilic moiety;
(ii) a plurality of amphiphilic molecules comprising at least one hydrophobic perfluorinated moiety linked to at least one hydrophilic metal complexing moiety comprising desferrioxamine or a derivative thereof.

**15.** A method for preparing a micelle composition, comprising a step of:

a) bringing into contact (**i**) a plurality of amphiphilic molecules comprising at least one hydrophobic perfluorinated moiety linked to at least one hydrophilic moiety, and (**ii**) a plurality of amphiphilic molecules comprising at least one hydrophobic perfluorinated moiety linked to at least one hydrophilic metal complexing moiety comprising desferrioxamine or a derivative thereof;
b) optionally ultra-sonicating the composition obtained at step a);

thereby preparing the micelle composition.

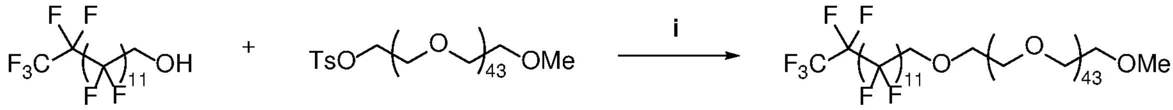


3

4

PFTD-PEG (1)

**FIGURE 1**

a)

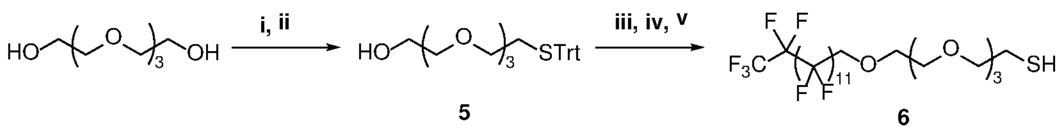


5

6

b)

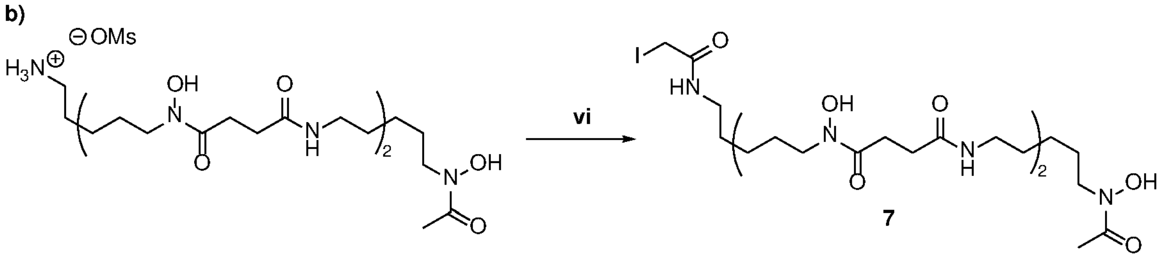


7

c)

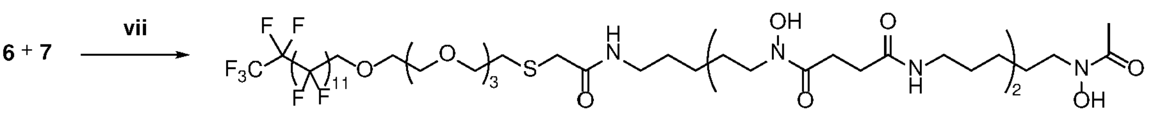


PFTD-DFO (2)

**FIGURE 2A, B, C**

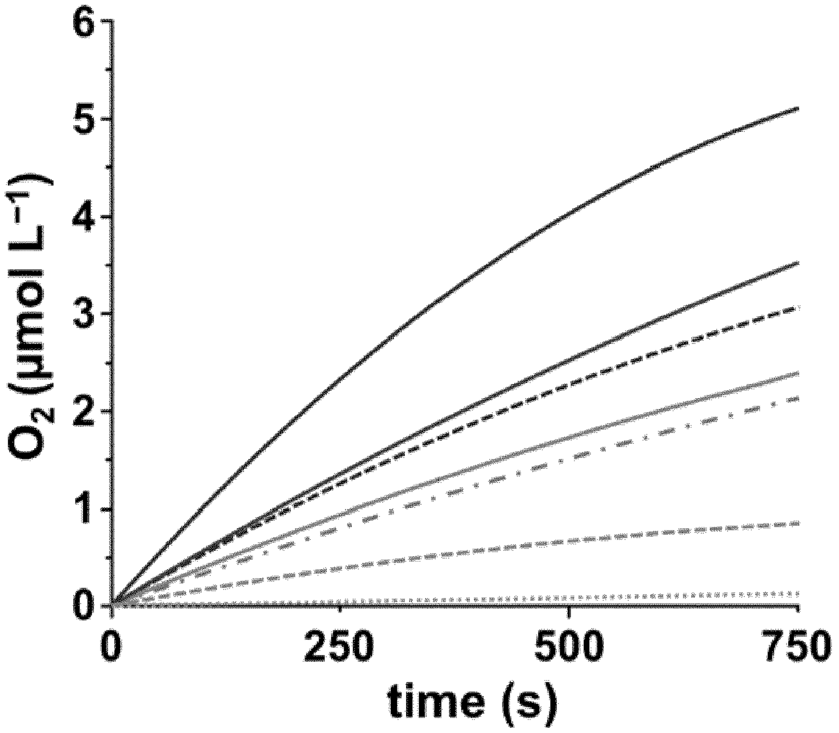


FIGURE 3A

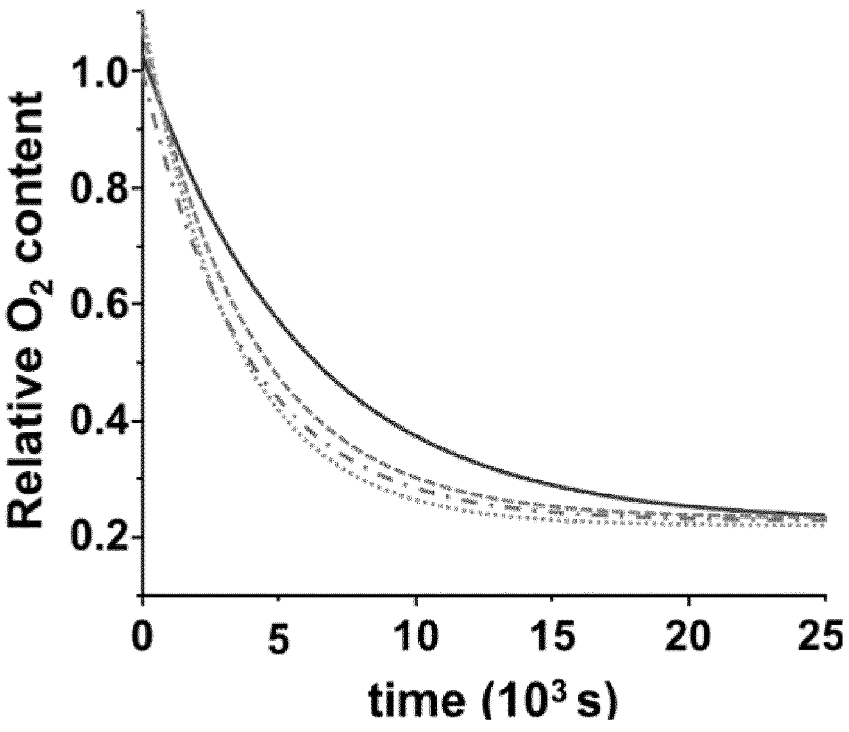


FIGURE 3B

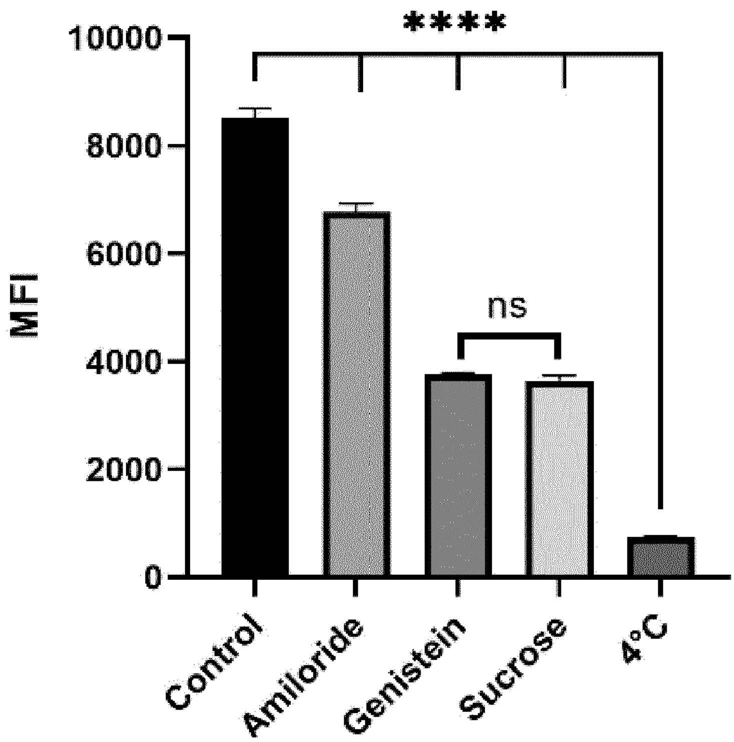
****
10000
8000
6000
4000
2000
0
MFI
ns
Control
Amiloride
Genistein
Sucrose
4°C

FIGURE 4

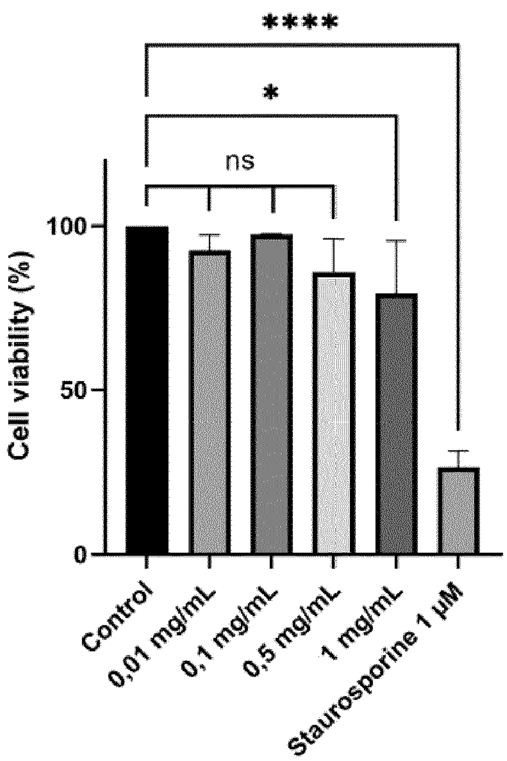


FIGURE 5A

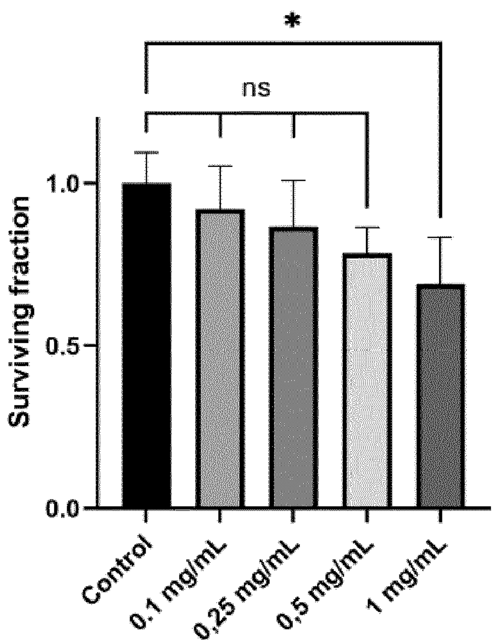


FIGURE 5B

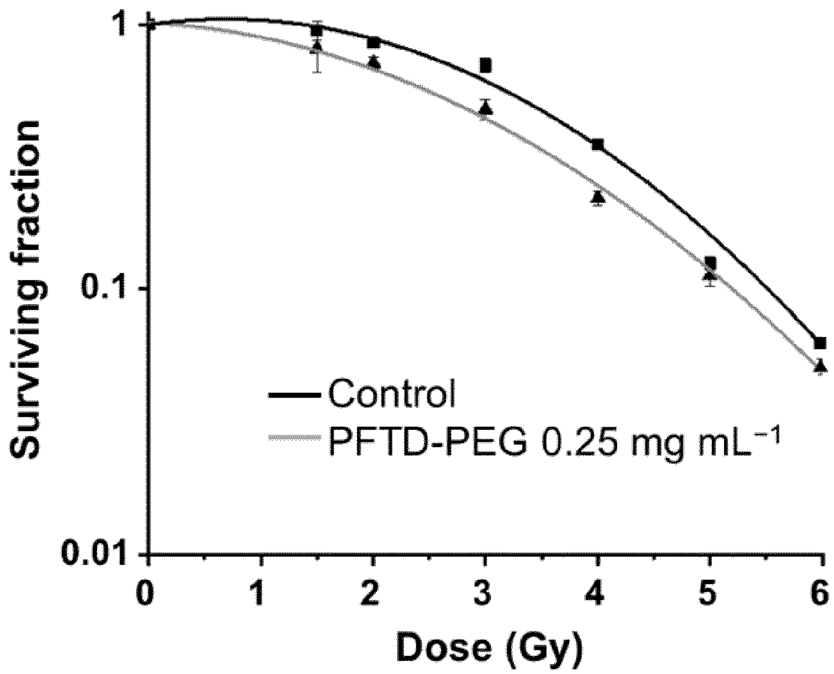
1
0.1
0.01
Surviving fraction
0
1
2
3
4
5
6
Dose (Gy)
Control
PFTD-PEG 0.25 mg mL⁻¹

FIGURE 6

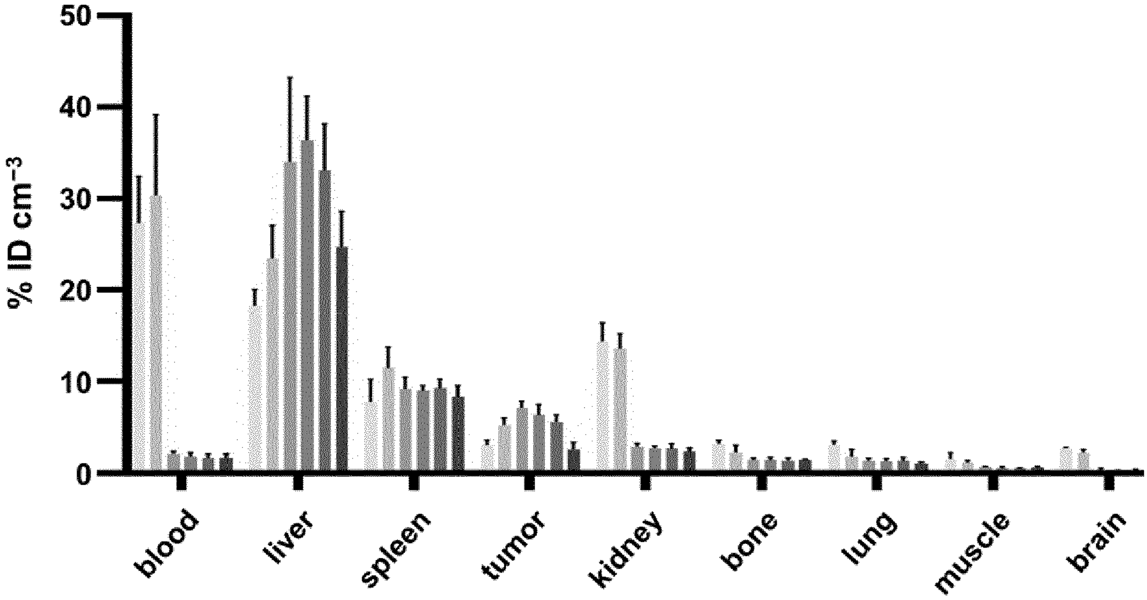
% ID cm−3
50
40
30
20
10
0
blood
liver
spleen
tumor
kidney
bone
lung
muscle
brain

FIGURE 7

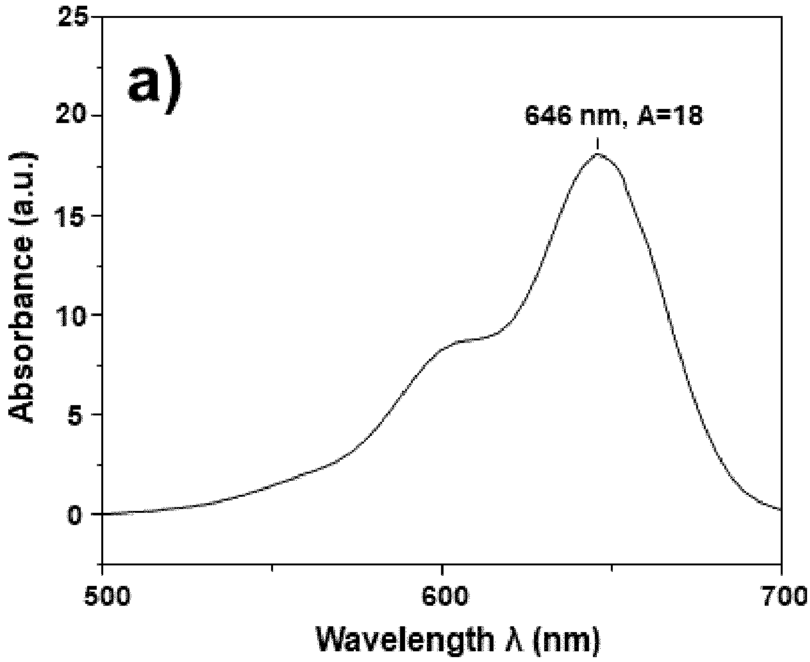


FIGURE 8A

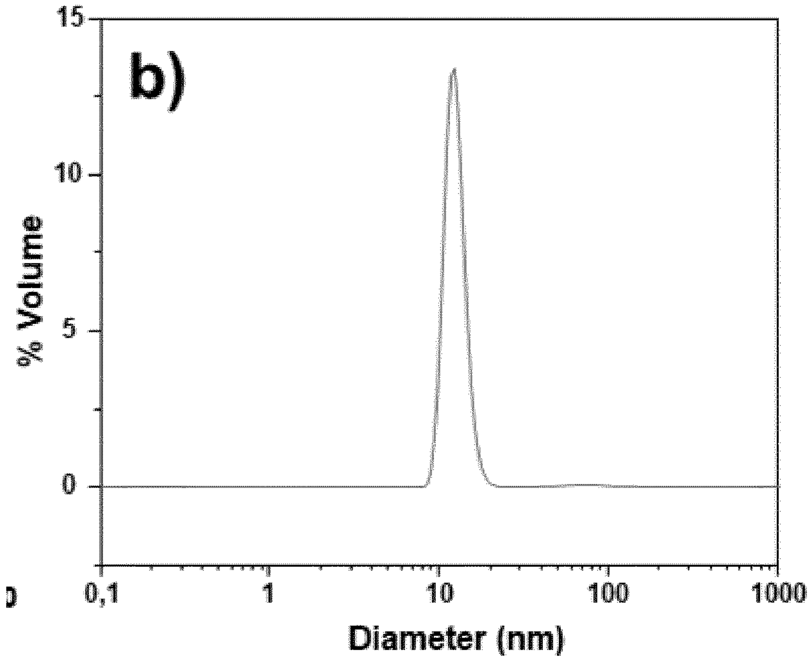


FIGURE 8B

Europäisches Patentamt
European Patent Office
Office européen des brevets

# EUROPEAN SEARCH REPORT

Application Number
EP 22 21 2442

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 484 622 A (SHANGHAI INST MATERIA MEDICA CAS) 4 August 2020 (2020-08-04)<br>* paragraph [0002] - paragraph [0005] *<br>* examples *<br>* figures *<br>* claims *<br>----- | 1-5 | INV.<br>A61K9/00<br>A61K9/107<br>A61K41/00<br>A61K51/04<br>A61K51/12<br>C07C323/52<br>A61K47/10<br>A61K47/20<br>C08G65/333<br>C08G65/334<br>C08G65/337 |
| X | YURONG QUE ET AL: "Enhancing Photodynamic Therapy Efficacy by Using Fluorinated Nanoplatform",<br>ACS MACRO LETTERS,<br>vol. 5, no. 2,<br>13 January 2016 (2016-01-13), pages 168-173, XP055690410,<br>ISSN: 2161-1653, DOI: 10.1021/acsmacrolett.5b00935<br>* the whole document *<br>* abstract *<br>* scheme 1 *<br>* table 1 *<br>* figures *<br>* penultimate paragraph;<br>page 169, right-hand column *<br>* page 172, left-hand column, last paragraph - right-hand column, paragraph 1 *<br>----- | 1-5 | TECHNICAL FIELDS SEARCHED (IPC)<br>A61K<br>C07C<br>C08G |
| A | EP 0 051 526 A1 (CENTRE NAT RECH SCIENT [FR]) 12 May 1982 (1982-05-12)<br>* examples *<br>* claims *<br>-----<br>-/-- | 1-7 | |

~~The present search report has been drawn up for all claims~~

| Place of search | Date of completion of the search | Examiner |
|---|---|---|
| The Hague | 8 June 2023 | Marchand, Petra |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 21 2442

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2010/077671 A2 (UNIV UTAH RES FOUND [US]; RAPOPORT NATALYA Y [US] ET AL.) 8 July 2010 (2010-07-08)<br>* page 1, line 8 - line 10 *<br>* page 8, line 17 - line 20 *<br>* examples *<br>* table 1 *<br>* claims *<br>----- | 1-7 | |
| A,D | WANG QIAN ET AL: "Fluorinated polymeric micelles to overcome hypoxia and enhance photodynamic cancer therapy", BIOMATERIALS SCIENCE, vol. 6, no. 11, 24 October 2018 (2018-10-24), pages 3096-3107, XP093050752, GB<br>ISSN: 2047-4830, DOI: 10.1039/C8BM00852C<br>* the whole document *<br>* abstract *<br>* figures *<br>* tables *<br>-----<br>-/-- | 1-7 | TECHNICAL FIELDS SEARCHED (IPC) |

~~The present search report has been drawn up for all claims~~

1

| Place of search | Date of completion of the search | Examiner |
|---|---|---|
| The Hague | 8 June 2023 | Marchand, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 21 2442

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | FAN MENG ET AL: "Increased Oxygen Solubility in Aqueous Media Using PEG-Poly-2,2,2-trifluoroethyl Methacrylate Copolymer Micelles and Their Potential Application As Volume Expanders and As an Artificial Blood Product", ACS APPLIED BIO MATERIALS, vol. 1, no. 3, 8 August 2018 (2018-08-08), pages 708-713, XP093050759, US ISSN: 2576-6422, DOI: 10.1021/acsabm.8b00173 * the whole document * * abstract * * scheme 1 * * figures * * conclusions * ----- | 1-7 | |
| | | | TECHNICAL FIELDS SEARCHED (IPC) |

~~The present search report has been drawn up for all claims~~

1

| Place of search | Date of completion of the search | Examiner |
|---|---|---|
| The Hague | 8 June 2023 | Marchand, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 22 21 2442

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**2-7(completely); 1(partially)**

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 22 21 2442

The Search Division considers that the present European patent application does not comply with the requirements of unity of invention and relates to several inventions or groups of inventions, namely:

**1. claims: 2-7(completely); 1(partially)**

**Biocompatible micelle composition, for use as a medicament, characterized in that: (i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons; (ii) the micelle has an average hydrodynamic diameter equal or inferior to 40 nm, wherein the micelles comprise an amphiphile of the structure CF3-CF2-(CF2)x-CH2-O-CH2-(CH2-O-Ch2)y-CH2-OMe, whereby x ranges from 8-15 and y ranges from 8 to 100, or x is 11 and y is 43. Further: compositions according to dependent claims 2-5**

---

**2. claims: 8-15(completely); 1(partially)**

**Biocompatible micelle composition, for use as a medicament, characterized in that: (i) the micelle comprises a plurality of amphiphilic molecules, said amphiphilic molecules comprising at least one hydrophobic perfluorinated moiety linked to at least one hydrophilic moiety, said amphiphilic molecules having a molecular weight of at least 500 Daltons; (ii) the micelle has an average hydrodynamic diameter equal or inferior to 40 nm, wherein the plurality of amphiphilic molecules comprises a hydrophobic perfluorinated moiety linked to a hydrophilic metal complexing moiety; in particular a hydrophobic metal complexing moiety comprising desferrioxamine of a derivative thereof: Further: Amphiphilic molecule according to claims 9-11. Method of claim 12, for preparing said amphiphilic molecule. Biocompatible micelle composition according to claim 13. Further: A kit according to claim 14. Further: A method as defined in claim 15 for preparing a micelle composition.**

---

# ANNEX TO THE EUROPEAN SEARCH REPORT ON EUROPEAN PATENT APPLICATION NO. EP 22 21 2442

This annex lists the patent family members relating to the patent documents cited in the above-mentioned European search report. The members are as contained in the European Patent Office EDP file on
The European Patent Office is in no way liable for these particulars which are merely given for the purpose of information.

08-06-2023

| Patent document cited in search report | | Publication date | Patent family member(s) | | Publication date |
|---|---|---|---|---|---|
| CN 111484622 | A | 04-08-2020 | NONE | | |
| EP 0051526 | A1 | 12-05-1982 | EP 0051526 | A1 | 12-05-1982 |
| | | | EP 0063149 | A1 | 27-10-1982 |
| | | | FR 2515198 | A1 | 29-04-1983 |
| | | | WO 8201467 | A1 | 13-05-1982 |
| WO 2010077671 | A2 | 08-07-2010 | EP 2373289 | A2 | 12-10-2011 |
| | | | US 2011306581 | A1 | 15-12-2011 |
| | | | WO 2010077671 | A2 | 08-07-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018187236 A
- WO 2010077671 A


### Non-patent literature cited in the description


- **BAUMANN et al.** *Nat. Rev. Cancer,* 2016, vol. 16, 234
- **SUWA et al.** *Exp. Mol. Med.,* 2021, vol. 53, 1029
- **WANG et al.** *Cancers,* 2019, vol. 11, 112
- **JAMGOTCHIAN et al.** *Nanoscale,* 2021, vol. 13, 2373
- **KRAFFT, M. P.** *Curr. Opin. Pharmacol.,* 2020, vol. 53, 117
- **JÄGERS et al.** *Eur. J. Physiol.,* 2021, vol. 473, 139-150
- **WANG et al.** *Biomater. Sci.,* 2019, vol. 6, 3096-3107
- **FAN et al.** *ACS Appl. Bio Mater.,* 2018, vol. 1, 708
- **PITARRESI et al.** *J. Fluor. Chem.,* 2008, vol. 129, 1096
- **BUTTERWORTH, K. T. et al.** *Nanoscale,* 2012, vol. 4, 4830
- **SUBIEL, A. et al.** *Theranostics,* 2016, vol. 6, 1651-1671